(19) Europäisches Patentamt
European Patent Office
Office européen des brevets

(11) **EP 4 624 592 A1**

(12) **EUROPEAN PATENT APPLICATION**
published in accordance with Art. 153(4) EPC

(43) Date of publication:
**01.10.2025 Bulletin 2025/40**

(21) Application number: **23910798.0**

(22) Date of filing: **28.12.2023**

(51) International Patent Classification (IPC):
*C12Q 1/6869* (2018.01)     *C12Q 1/6888* (2018.01)
*G16B 30/10* (2019.01)

(86) International application number:
**PCT/CN2023/142606**

(87) International publication number:
**WO 2024/140881 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.12.2022 CN 202211727280**

(71) Applicant: **GeneMind Biosciences Company
Limited
Shenzhen, Guangdong 518000 (CN)**

(72) Inventors:
• **CHEN, Weiyue
Shenzhen, Guangdong 518000 (CN)**
• **ZHANG, Yanhua
Shenzhen, Guangdong 518000 (CN)**
• **LIU, Xianke
Shenzhen, Guangdong 518000 (CN)**
• **ZHANG, Juan
Shenzhen, Guangdong 518000 (CN)**

(74) Representative: **Schiweck Weinzierl Koch
Patentanwälte Partnerschaft mbB
Ganghoferstraße 68 B
80339 München (DE)**

(54) **METHOD AND DEVICE FOR DETERMINING FETAL DNA CONCENTRATION**

(57)     Provided are a method and apparatus for determining a concentration of fetal DNA. The method for determining the concentration of fetal DNA includes: acquiring multiple sequencing reads of a cell-free DNA (cfDNA) sample under test; aligning the multiple sequencing reads with a reference genome to obtain alignment results; and determining the concentration of fetal DNA based on the alignment results. The concentration of fetal DNA can be determined based on the alignment results between the sequencing reads and the reference genome so that applications to various sequencing platforms including a single-molecule sequencing platform can be satisfied, and the accuracy of determination of the concentration of fetal DNA can be improved.

FIG. 1

**Description**

This application claims priority to Chinese Patent Application No. 202211727280.7 titled FETAL CONCENTRATION DETERMINATION METHOD AND APPARATUS and filed with the China National Intellectual Property Administration (CNIPA) on Dec. 30, 2022, the disclosure of which is incorporated herein by reference in its entirety.

TECHNICAL FIELD

The present application relates to the field of bioinformation technology and, in particular, to a method and apparatus for determining a concentration of fetal DNA.

BACKGROUND

In noninvasive prenatal testing (NIPT), the concentration of fetal DNA refers to a proportion of fetal deoxyribonucleic acid (DNA) in the peripheral blood cell-free DNA (cfDNA) of a pregnant woman. The concentration of fetal DNA is an important parameter in the NIPT.

For second-generation sequencing, currently there are methods for evaluating the concentration of fetal DNA. For example, the concentration of fetal DNA is determined by using the distribution characteristics of cfDNA lengths, methylation characteristics, single-nucleotide polymorphism (SNP) characteristics, or the contents of chromosome X and chromosome Y in a male fetus. Third-generation sequencing is single-molecule sequencing. The sequencing principle of the third-generation sequencing is different from that of the second-generation sequencing, and data features generated in the third-generation sequencing have relatively large differences from those generated in the second-generation sequencing. Therefore, the methods for evaluating the concentration of fetal DNA, which are applicable to the second-generation sequencing, are not applicable to third-generation sequencing platforms. Therefore, it is very important to find a method for evaluating the concentration of fetal DNA, which is applicable to both the second-generation sequencing and the third-generation sequencing.

SUMMARY

The sequencing principle of third-generation sequencing is different from that of second-generation sequencing, and data features generated in the third-generation sequencing are different from the features of sequencing data obtained in the second-generation sequencing. These differences lead to some differences in applications of the third-generation sequencing and the second-generation sequencing. For example, since the length of an inserted sequence cannot be measured by a third-generation sequencing platform, the concentration of fetal DNA cannot be determined by using the distribution characteristics of cfDNA lengths. For example, since methylation information cannot be measured by the third-generation sequencing platform, the concentration of fetal DNA cannot be determined by extracting methylation characteristics. For example, due to a difference in the sequencing principle of the third-generation sequencing platform, error features generated in the sequencing process of the third-generation sequencing platform are different from error features generated in the second-generation sequencing; therefore, the SNP method applicable to the second-generation sequencing cannot be applied to the determination of the concentration of fetal DNA based on the third-generation sequencing platform. The determination of the concentration of fetal DNA by using the contents of chromosome X and chromosome Y can only be performed on the premise of a male fetus and cannot be applied to a female fetus. Therefore, based on the preceding cases, it is necessary to develop a method for evaluating the concentration of fetal DNA, which is compatible with various sequencing platforms and applicable to fetuses of different genders.

In view of this, the present application provides the technical solutions below.

A method for determining a concentration of fetal DNA includes the steps below.

Multiple sequencing reads of a cfDNA sample under test are acquired.

The multiple sequencing reads are aligned with a reference genome to obtain alignment results.

The concentration of fetal DNA is determined based on the alignment results.

As a possible implementation of the present application, that the multiple sequencing reads are aligned with the reference genome to obtain the alignment results includes the steps below.

The reference genome is segmented into multiple segment bins.

A first alignment count of sequencing reads falling within each segment bin of the multiple segment bins is determined among the multiple sequencing reads, and first alignment counts of the multiple segment bins are determined as the alignment results.

As a possible implementation of the present application, that the reference genome is segmented into the multiple segment bins includes the steps below.

Based on a preset segmentation length, the reference genome is segmented into multiple initial segment bins.

Segment bins corresponding to a particular chromosome are removed from the multiple initial segment bins to obtain the multiple segment bins.

Alternatively, a particular chromosome is removed from the reference genome to obtain a removed reference genome.

Based on a preset segmentation length, the removed reference genome is segmented into the multiple segment bins.

As a possible implementation of the present application, the particular chromosome includes at least one of chromosome X, chromosome Y, a mitochondrial chromosome, chromosome 13, chromosome 18, or chromosome 21.

As a possible implementation of the present application, the method further includes the step below.

The first alignment count is corrected to obtain a corrected first alignment count, and corrected first alignment counts of the multiple segment bins are determined as the alignment results.

As a possible implementation of the present application, that the first alignment count is corrected to obtain the corrected first alignment count includes the steps below.

Based on the first alignment count of each segment bin and a mean of the first alignment counts of the multiple segment bins, the first alignment count of each segment bin is normalized to obtain a normalized first alignment count of each segment bin.

Guanine-cytosine (GC) correction is performed on the normalized first alignment count of each segment bin to obtain a GC-corrected first alignment count.

As a possible implementation of the present application, that the GC correction is performed on the normalized first alignment count of each segment bin to obtain the GC-corrected first alignment count includes the steps below.

A first relationship curve is generated based on a GC content and the normalized first alignment count of each segment bin.

Based on the first relationship curve, the GC correction is performed on the normalized first alignment count to obtain the GC-corrected first alignment count.

As a possible implementation of the present application, before the first relationship curve is generated based on the GC content and the normalized first alignment count of each segment bin, the method further includes the step below.

Each segment bin is filtered based on the GC content of each segment bin to enable the first relationship curve to be generated based on the GC content of each segment bin after GC filtering and the normalized first alignment count of each segment bin.

Alternatively, that based on the first relationship curve, the GC correction is performed on the normalized first alignment count to obtain the GC-corrected first alignment count includes the step below.

Each segment bin is filtered based on the GC content of each segment bin, and based on the first relationship curve, the GC correction is performed on the normalized first alignment count of each segment bin after GC filtering to obtain the GC-corrected first alignment count.

As a possible implementation of the present application, the GC correction involves determining the GC-corrected first alignment count according to a subtraction or division relation between the normalized first alignment count of each segment bin and the GC content of each segment bin.

As a possible implementation of the present application, that the first alignment count is corrected to obtain the corrected first alignment count further includes the steps below.

The reference genome is cut based on a particular sliding window length to obtain sequence cuts, the sequence cuts are aligned with the reference genome, a first alignment count of sequence cuts falling within each segment bin is counted, and the first alignment count is determined as a sliding window alignment count of each segment bin.

Based on the sliding window alignment count, a normalized sliding window alignment count of each segment bin is determined.

Alignment probability correction is performed based on the normalized sliding window alignment count and the normalized first alignment count of each segment bin to determine a first alignment count subjected to the alignment probability correction.

As a possible implementation of the present application, that the alignment probability correction is performed based on the normalized sliding window alignment count and the normalized first alignment count of each segment bin to determine the first alignment count subjected to the alignment probability correction includes the steps below.

A second relationship curve is generated based on the normalized sliding window alignment count of each segment bin and the normalized first alignment count corresponding to each segment bin.

Based on the second relationship curve, the alignment probability correction is performed on the normalized first alignment count to obtain the first alignment count subjected to the alignment probability correction.

As a possible implementation of the present application, before the second relationship curve is generated based on the normalized sliding window alignment count of each segment bin and the normalized first alignment count corresponding to each segment bin, the method further includes the step below.

The multiple segment bins are filtered based on normalized sliding window alignment counts of the multiple segment bins, and segment bins whose normalized sliding window alignment counts are each not less than a first target threshold are obtained to enable the second relationship curve to be generated for the segment bins whose normalized sliding window alignment counts are each not less than the first target threshold.

Alternatively, that based on the second relationship curve, the alignment probability correction is performed on the normalized first alignment count to obtain the first alignment count subjected to the alignment probability correction includes the step below.

The multiple segment bins are filtered based on normalized sliding window alignment counts of the multiple segment bins, and second relationship curve sections of the second relationship curve where normalized sliding window alignment counts are each not less than a first target threshold are retained to obtain a second relationship curve after alignment probability filtering.

Based on the second relationship curve after alignment probability filtering, the alignment probability correction is performed on the normalized sliding window alignment count to obtain the first alignment count subjected to the alignment probability correction.

As a possible implementation of the present application, that the concentration of fetal DNA is determined based on the alignment results includes the steps below.

First training sample data are obtained, where each sample in the first training sample data is labeled with first feature values and a first target value, the first feature values are sample alignment counts, and the first target value is an actual concentration of fetal DNA.

Based on a particular model structure, machine learning modeling is performed on the first training sample data to obtain a first concentration quantitation model of fetal DNA.

The alignment results are input into the first concentration quantitation model of fetal DNA to obtain a first concentration of fetal DNA and the first concentration of fetal DNA is determined as the concentration of fetal DNA.

As a possible implementation of the present application, that based on the particular model structure, the machine learning modeling is performed on the first training sample data to obtain the first concentration quantitation model of fetal DNA includes the steps below.

The first training sample data are divided into a training set and a test set.

The machine learning modeling is performed based on the training set to obtain an initial model.

The test set is processed based on the initial model to obtain a predicted concentration of fetal DNA in each test sample in the test set.

The predicted concentration of fetal DNA is compared with an actual concentration of fetal DNA in each test sample in the test set to obtain a comparison result.

A model parameter of the initial model is adjusted based on the comparison result to obtain the first concentration quantitation model of fetal DNA.

As a possible implementation of the present application, that the concentration of fetal DNA is determined based on the alignment results includes the steps below.

The alignment results are input into a first preset model to obtain an initial concentration of fetal DNA, where the first preset model is a linear relationship model built based on alignment results in second sample data and an initial concentration of fetal DNA corresponding to the alignment results, and the second sample data include the alignment results of alignment of sequencing reads of a cfDNA sample with the reference genome and the concentration of fetal DNA corresponding to the alignment results.

The initial concentration of fetal DNA is corrected based on a second preset model to obtain the concentration of fetal DNA, where the second preset model is a model obtained after the first preset model is processed based on constants determined through linear fitting.

As a possible implementation of the present application, that the sequencing reads are aligned with the reference genome to obtain the alignment results includes the steps below.

A second alignment count of times each base site of the reference genome acts as starting positions of alignment of sequencing reads with the reference genome is determined, and based on the second alignment count, a nucleosome center score corresponding to each base site of the reference genome is calculated.

Nucleosome center positions are determined based on nucleosome center scores corresponding to base sites of the reference genome and a center score screening threshold.

Based on the nucleosome center positions, alignment and summation are performed on second alignment counts at corresponding positions within all nucleosome regions to obtain summed second alignment counts.

Dimensionality reduction is performed on the summed second alignment counts to obtain normalized second alignment counts subjected to the dimensionality reduction, and the normalized second alignment counts subjected to the dimensionality reduction are determined as the alignment results.

As a possible implementation of the present application, that the nucleosome center score corresponding to each base site of the reference genome is calculated includes the steps below.

A first count mean of second alignment counts of a first particular number of bases on the left and the first particular number of bases on the right of each base site of the reference genome is calculated.

A second count mean of second alignment counts of a second particular number of bases on the left and the second particular number of bases on the right of each base site of the reference genome is calculated.

The nucleosome center score corresponding to each base site is determined according to the first count mean and the second count mean.

As a possible implementation of the present application, the nucleosome center score is calculated by the following formula:

$$\text{the nucleosome center score} =$$

$$\frac{count\ mean\ in\ abin[x-93,x-74+n]+count\ mean\ in\ a\ bin\ [x+93,x+74-n]}{count\ mean\ in\ a\ bin[x-73+n,x+73-n]},$$

where $x$ represents the base site, [x-93, x-74+n] represents a bin from a site 93 nucleotides away from $x$ on a side of $x$ to a site (74-n) nucleotides away from $x$ on the same side, [x+93, x+74-n] represents a bin from a site 93 nucleotides away from $x$ on the other side of $x$ to a site (74-$n$) nucleotides away from $x$ on the same side, [$x$-73+$n$, $x$+73-$n$] represents a bin from a site (73-n) nucleotides away from $x$ on the side of $x$ to a site (73-n) nucleotides away from $x$ on the other side of x, and n represents a natural number less than or equal to 5.

As a possible implementation of the present application, that the nucleosome center scores are determined based on the nucleosome center scores corresponding to the base sites of the reference genome and the center score screening threshold includes the steps below.

A maximum value of the nucleosome center scores is determined and a first position corresponding to the maximum value in the reference genome is determined.

Nucleosome center scores of bases of particular data on two sides of the first position are zeroed, a maximum value is re-determined from the remaining nucleosome center scores after zeroing, and a position corresponding to the re-determined maximum value in the reference genome is determined, until the remaining nucleosome center scores are each less than a second target threshold, and screened positions are determined as candidate nucleosome center positions.

The nucleosome center positions are determined based on the center score screening threshold and nucleosome center scores corresponding to the candidate nucleosome center positions.

As a possible implementation of the present application, in the step of, based on the nucleosome center positions, performing the alignment and the summation on the second alignment counts at the corresponding positions within all the nucleosome regions, the corresponding positions within each nucleosome region include a particular number of bases on the left and the particular number of bases on the right of each of the nucleosome center positions.

As a possible implementation of the present application, that the concentration of fetal DNA is determined based on the alignment results includes the steps below.

Second training sample data are acquired, where each sample in the second training sample data is labeled with feature values and a target value, the feature values are normalized second alignment counts subjected to the dimensionality reduction, and the target value is an actual concentration of fetal DNA.

Based on the second training sample data, a second concentration quantitation model of fetal DNA is built.

The alignment results are input into the second concentration quantitation model of fetal DNA to obtain the concentration of fetal DNA.

A method for determining a concentration of fetal DNA includes the steps below.

Multiple sequencing reads of a cfDNA sample under test are acquired.

A reference genome is segmented into multiple segment bins, and a first alignment count of sequencing reads falling within each segment bin of the multiple segment bins is determined among the multiple sequencing reads.

A first concentration of fetal DNA is determined based on first alignment counts of the multiple segment bins.

Based on a second alignment count of times each base site of the reference genome acts as starting positions of alignment of sequencing reads with the reference genome, nucleosome center positions are determined.

Based on the nucleosome center positions, alignment and summation are performed on second alignment counts at corresponding positions within all nucleosome regions to obtain summed second alignment counts.

Alignment results are determined based on the first concentration of fetal DNA and the summed second alignment counts.

The concentration of fetal DNA is determined based on the alignment results.

As a possible implementation of the present application, that the reference genome is segmented into the multiple segment bins includes the steps below.

Based on a preset segmentation length, the reference genome is segmented into multiple initial segment bins.

Segment bins corresponding to a particular chromosome are removed from the multiple initial segment bins to obtain the multiple segment bins.

Alternatively, a particular chromosome is removed from the reference genome to obtain a removed reference genome.

Based on a preset segmentation length, the removed reference genome is segmented into the multiple segment bins.

As a possible implementation of the present application, the particular chromosome includes at least one of chromosome X, chromosome Y, a mitochondrial chromosome, chromosome 13, chromosome 18, or chromosome 21.

As a possible implementation of the present application, before the first concentration of fetal DNA is determined based on the first alignment counts of the multiple segment bins, the method further includes the step below.

The first alignment count is corrected to obtain a corrected first alignment count, and the first concentration of fetal DNA is determined based on corrected first alignment counts of the multiple segment bins.

As a possible implementation of the present application, that the first alignment count is corrected to obtain the corrected first alignment count includes the steps below.

Based on the first alignment count of each segment bin and a mean of the first alignment counts of the multiple segment bins, the first alignment count of each segment bin is normalized to obtain a normalized first alignment count of each segment bin.

GC correction is performed on the normalized first alignment count of each segment bin to obtain a GC-corrected first alignment count.

As a possible implementation of the present application, that the GC correction is performed on the normalized first alignment count of each segment bin to obtain the GC-corrected first alignment count includes the steps below.

A first relationship curve matching a particular coordinate system is generated based on a GC content and the normalized first alignment count of each segment bin.

Based on the first relationship curve, the GC correction is performed on the normalized first alignment count to obtain the GC-corrected first alignment count.

As a possible implementation of the present application, before the first relationship curve is generated based on the GC content and the normalized first alignment count of each segment bin, the method further includes the step below.

Each segment bin is filtered based on the GC content of each segment bin to enable the first relationship curve to be generated based on the GC content of each segment bin after GC filtering and the normalized first alignment count of each segment bin.

Alternatively, that based on the first relationship curve, the GC correction is performed on the normalized first alignment count to obtain the GC-corrected first alignment count includes the step below.

Each segment bin is filtered based on the GC content of each segment bin, and based on the first relationship curve, the GC correction is performed on the normalized first alignment count of each segment bin after GC filtering to obtain the GC-corrected first alignment count.

As a possible implementation of the present application, the GC correction involves determining the GC-corrected first alignment count according to a subtraction or division relation between the normalized first alignment count of each segment bin and the GC content of each segment bin.

As a possible implementation of the present application, that the first alignment count is corrected to obtain the corrected first alignment count further includes the steps below.

The reference genome is cut based on a particular sliding window length to obtain sequence cuts, the sequence cuts are aligned with the reference genome, a first alignment count of sequence cuts falling within each segment bin is counted, and the first alignment count is determined as a sliding window alignment count of each segment bin.

Based on the sliding window alignment count, a normalized sliding window alignment count of each segment bin is determined.

Alignment probability correction is performed based on the normalized sliding window alignment count and the normalized first alignment count of each segment bin to determine a first alignment count subjected to the alignment probability correction.

As a possible implementation of the present application, that the alignment probability correction is performed based on the normalized sliding window alignment count and the normalized first alignment count of each segment bin to determine the first alignment count subjected to the alignment probability correction includes the steps below.

A second relationship curve is generated based on the normalized sliding window alignment count of each segment bin and the normalized first alignment count corresponding to each segment bin.

Based on the second relationship curve, the alignment probability correction is performed on the normalized first alignment count to obtain the first alignment count subjected to the alignment probability correction.

As a possible implementation of the present application, before the second relationship curve is generated based on the normalized sliding window alignment count of each segment bin and the normalized first alignment count corresponding to each segment bin, the method further includes the step below.

The multiple segment bins are filtered based on normalized sliding window alignment counts of the multiple segment bins, and segment bins whose normalized sliding window alignment counts are each not less than a first target threshold are obtained to enable the second relationship curve to be generated for the segment bins whose normalized sliding window alignment counts are each not less than the first target threshold.

Alternatively, that based on the second relationship curve, the alignment probability correction is performed on the normalized first alignment count to obtain the first alignment count subjected to the alignment probability correction includes the step below.

The multiple segment bins are filtered based on normalized sliding window alignment counts of the multiple segment bins, and second relationship curve sections of the second relationship curve where normalized sliding window alignment counts are each not less than a first target threshold are retained to obtain a second relationship curve after alignment probability filtering.

Based on the second relationship curve after alignment probability filtering, the alignment probability correction is performed on the normalized sliding window alignment count to obtain the first alignment count subjected to the alignment probability correction.

As a possible implementation of the present application, that the first concentration of fetal DNA is determined based on the first alignment counts of the multiple segment bins includes the steps below.

First training sample data are obtained, where each sample in the first training sample data is labeled with first feature values and a first target value, the first feature values are alignment counts, and the first target value is an actual concentration of fetal DNA.

Based on a particular model structure, machine learning modeling is performed on the first training sample data to obtain a first concentration quantitation model of fetal DNA.

The first alignment counts are input into the first concentration quantitation model of fetal DNA to obtain the first concentration of fetal DNA.

As a possible implementation of the present application, that based on the particular model structure, the machine learning modeling is performed on the first training sample data to obtain the first concentration quantitation model of fetal DNA includes the steps below.

The first training sample data are divided into a training set and a test set.

The machine learning modeling is performed based on the training set to obtain an initial model.

The test set is processed based on the initial model to obtain a predicted concentration of fetal DNA in each test sample in the test set.

The predicted concentration of fetal DNA is compared with an actual concentration of fetal DNA in each test sample in the test set to obtain a comparison result.

A model parameter of the initial model is adjusted based on the comparison result to obtain the first concentration quantitation model of fetal DNA.

As a possible implementation of the present application, that the first concentration of fetal DNA is determined based on the first alignment counts of the multiple segment bins includes the steps below.

The first alignment counts are input into a first preset model to obtain an initial concentration of fetal DNA, where the first preset model is a linear relationship model built based on alignment results in second sample data and an initial concentration of fetal DNA corresponding to the alignment results, and the second sample data include the alignment results of alignment of sequencing reads of a cfDNA sample with the reference genome and the concentration of fetal DNA corresponding to the alignment results.

The initial concentration of fetal DNA is corrected based on a second preset model to obtain the first concentration of fetal DNA, where the second preset model is a model obtained after the first preset model is processed based on constants determined through linear fitting.

As a possible implementation of the present application, that based on the second alignment count of times each base site of the reference genome acts as the starting positions of alignment of the sequencing reads with the reference genome, the nucleosome center positions are determined includes the steps below.

The second alignment count of times each base site of the reference genome acts as the starting positions of alignment of the sequencing reads with the reference genome is determined, and based on the second alignment count, a nucleosome center score corresponding to each base site of the reference genome is calculated.

The nucleosome center positions are determined based on nucleosome center scores corresponding to base sites of the reference genome and a center score screening threshold.

As a possible implementation of the present application, that the alignment results are determined based on the first concentration of fetal DNA and the summed second alignment counts includes the steps below.

Dimensionality reduction is performed on the summed second alignment counts corresponding to base sites within the nucleosome regions to obtain normalized second alignment counts subjected to the dimensionality reduction.

The alignment results are determined based on the first concentration of fetal DNA and the normalized second alignment counts subjected to the dimensionality reduction.

Alternatively, initial alignment results are determined based on the first concentration of fetal DNA and the summed second alignment counts.

Dimensionality reduction is performed on the initial alignment results, and initial alignment results subjected to the dimensionality reduction are determined as the alignment results.

As a possible implementation of the present application, that the nucleosome center score corresponding to each base site of the reference genome is calculated includes the steps below.

A first count mean of second alignment counts of a first particular number of bases on the left and the first particular number of bases on the right of each base site of the reference genome is calculated.

A second count mean of second alignment counts of a second particular number of bases on the left and the second particular number of bases on the right of each base site of the reference genome is calculated.

The nucleosome center score corresponding to each base site is determined according to the first count mean and the second count mean.

As a possible implementation of the present application, the nucleosome center score is calculated by the following formula:

$$\text{the nucleosome center score} =$$

$$\frac{count\ mean\ in\ a\ bin[x-93,x-74+n]+count\ mean\ in\ a\ bin\ [x+93,x+74-n]}{count\ mean\ in\ a\ bin[x-73+n,x+73-n]},$$

where $x$ represents the base site, [x-93, x-74+n] represents a bin from a site 93 nucleotides away from $x$ on a side of $x$ to a site (74-n) nucleotides away from $x$ on the same side, [x+93, $x$+74-n] represents a bin from a site 93 nucleotides away from $x$ on the other side of $x$ to a site (74-$n$) nucleotides away from $x$ on the same side, [x-73+n, $x$+73-n] represents a bin from a site (73-$n$) nucleotides away from $x$ on the side of $x$ to a site (73-$n$) nucleotides away from $x$ on the other side of x, and n represents a natural number less than or equal to 5.

As a possible implementation of the present application, that the nucleosome center scores are determined based on the nucleosome center scores corresponding to the base sites of the reference genome and the center score screening threshold includes the steps below.

A maximum value of the nucleosome center scores is determined and a first position corresponding to the maximum value in the reference genome is determined.

Nucleosome center scores of bases of particular data on two sides of the first position are zeroed, a maximum value is re-determined from the remaining nucleosome center scores after zeroing, and a position corresponding to the re-determined maximum value in the reference genome is determined, until the remaining nucleosome center scores are each less than a second target threshold, and screened positions are determined as candidate nucleosome center positions.

The nucleosome center positions are determined based on the center score screening threshold and nucleosome center scores corresponding to the candidate nucleosome center positions.

As a possible implementation of the present application, in the step of, based on the nucleosome center positions, performing the alignment and the summation on the second alignment counts at the corresponding positions within all the nucleosome regions, the corresponding positions within each nucleosome region include a particular number of bases on the left and the particular number of bases on the right of each of the nucleosome center positions.

As a possible implementation of the present application, that the concentration of fetal DNA is determined based on the alignment results includes the steps below.

Second training sample data are acquired, where each sample in the second training sample data is labeled with feature values and a target value, the feature values are normalized second alignment counts subjected to the dimensionality reduction, and the target value is an actual concentration of fetal DNA.

Based on the second training sample data, a second concentration quantitation model of fetal DNA is built.

The alignment results are input into the second concentration quantitation model of fetal DNA to obtain the concentration of fetal DNA.

An apparatus for determining a concentration of fetal DNA includes a first acquisition unit, a first alignment unit, and a first determination unit.

The first acquisition unit is configured to acquire multiple sequencing reads of a cfDNA sample under test.

The first alignment unit is configured to align the multiple sequencing reads with a reference genome to obtain alignment results.

The first determination unit is configured to determine the concentration of fetal DNA based on the alignment results.

An apparatus for determining a concentration of fetal DNA includes a second acquisition unit, a second determination unit, a third determination unit, a fourth determination unit, a processing unit, a fifth determination unit, and a sixth determination unit.

The second acquisition unit is configured to acquire multiple sequencing reads of a cfDNA sample under test.

The second determination unit is configured to segment a reference genome into multiple segment bins and determine, among the multiple sequencing reads, a first alignment count of sequencing reads falling within each segment bin of the multiple segment bins.

The third determination unit is configured to determine a first concentration of fetal DNA based on first alignment counts of the multiple segment bins.

The fourth determination unit is configured to, based on a second alignment count of times each base site of the reference genome acts as starting positions of alignment of sequencing reads with the reference genome, determine nucleosome center positions.

The processing unit is configured to, based on the nucleosome center positions, perform alignment and summation on second alignment counts at corresponding positions within all nucleosome regions to obtain summed second alignment counts.

The fifth determination unit is configured to determine alignment results based on the first concentration of fetal DNA and the summed second alignment counts.

The sixth determination unit is configured to determine the concentration of fetal DNA based on the alignment results.

As can be known from the preceding technical solutions, the present application discloses the method and apparatus for determining the concentration of fetal DNA. The method includes: acquiring the multiple sequencing reads of the cfDNA sample under test; aligning the multiple sequencing reads with the reference genome to obtain the alignment results; and determining the concentration of fetal DNA based on the alignment results. In the present application, the concentration of fetal DNA can be determined based on the alignment results between the sequencing reads and the reference genome so that applications to various sequencing platforms including a single-molecule sequencing platform can be satisfied, and the accuracy of determination of the concentration of fetal DNA can be improved. Additionally, the method for determining the concentration of fetal DNA according to the present application does not rely on the identification and confirmation of gender-dependent features. Therefore, the method is applicable to the determination of the concentration of fetal DNA of both the male fetus and the female fetus, making up for the deficiency that the third-generation sequencing platform cannot effectively evaluate the concentration of fetal DNA of the female fetus.

BRIEF DESCRIPTION OF DRAWINGS

To illustrate the technical solutions in embodiments of the present application more clearly, drawings used in the description of the embodiments are briefly described below. Apparently, the drawings described below illustrate the embodiments of the present application, and those of ordinary skill in the art can obtain other drawings based on the drawings described below on the premise that no creative work is done.

FIG. 1 is a flowchart of a method for determining a concentration of fetal DNA according to an embodiment of the present application.

FIG. 2 is a schematic diagram of a relationship between concentrations of fetal DNA calculated by an elastic net (Enet) model and reference concentrations of fetal DNA according to an embodiment of the present application.

FIG. 3 is a schematic diagram of a relationship between concentrations of fetal DNA calculated by a weight rank selection criterion (WRSC) model and reference concentrations of fetal DNA according to an embodiment of the present application.

FIG. 4 is a schematic diagram of a relationship between concentrations of fetal DNA calculated according to original parameters of gmFF_V7 and reference concentrations of fetal DNA according to an embodiment of the present application.

FIG. 5 is a schematic diagram of a relationship between concentrations of fetal DNA calculated by gmFF_V7 after linear correction and reference concentrations of fetal DNA according to an embodiment of the present application.

FIG. 6 is a schematic diagram of the distribution of a set of data subjected to alignment and summation according to nucleosome regions according to an embodiment of the present application.

FIG. 7 is a schematic diagram of a prediction effect of a model after random division into a training set and a test set according to an embodiment of the present application.

FIG. 8 is a flowchart of another method for determining a concentration of fetal DNA according to an embodiment of the present application.

FIG. 9 is a structural diagram of an apparatus for determining a concentration of fetal DNA according to an embodiment of the present application.

FIG. 10 is a structural diagram of another apparatus for determining a concentration of fetal DNA according to an embodiment of the present application.

DETAILED DESCRIPTION

The technical solutions of the present application are described clearly and completely below in conjunction with embodiments of the present application and the drawings thereof. Apparently, the described embodiments are merely part, not all, of embodiments of the present application. Based on the embodiments of the present application, all other embodiments obtained by those of ordinary skill in the art without creative work are within the scope of the present application.

Terms such as "first" and "second" in the description, claims, and drawings of the present application are used to distinguish between different objects and are neither used to describe a particular order nor construed as indicating or implying relative importance or implicitly indicating the number of technical features indicated. For example, without departing from the scope of the embodiments of the present application, the expressions of "first alignment count" and "second alignment count" in the present application are interchangeable.

Terms "including", "having", and any variations thereof are intended to encompass a non-exclusive inclusion. For example, a process, method, system, product, or device that includes a series of steps or units is not limited to the listed steps or units and may include other steps or units that are not listed.

The term "sequencing" may also be referred to as "nucleic acid sequencing" or "gene sequencing", that is, the three expressions are interchangeable. The sequencing refers to the determination of types and an arrangement order of bases in a nucleic acid sequence. The sequencing includes sequencing by synthesis (SBS) and/or sequencing by ligation (SBL), includes DNA sequencing and/or RNA sequencing, or includes long fragment sequencing and/or short fragment sequencing. A long fragment and a short fragment are relative, for example, a nucleic acid molecule of longer than 1 kb, 2 kb, 5 kb, or 10 kb may be referred to as a long fragment and a nucleic acid molecule of shorter than 1 kb or 800 bp may be referred to as a short fragment.

The term "single-molecule sequencing" refers to a single-copy sequencing technology based on a base signal collection unit, that is, a technology for directly reading a sequence of a nucleic acid molecule under test without performing signal amplification on the nucleic acid molecule by using a particular amplification technology. The term "single-molecule sequencing platform" refers to a sequencing platform based on the single-molecule sequencing and includes various sequencers based on the single-molecule sequencing, including, but not limited to, a GenoCare sequencing platform of GeneMind.

Embodiments of the present application provide a method for determining a concentration of fetal DNA. The method is applicable to the evaluation of the concentration of fetal DNA based on various sequencing platforms. In particular, a method for evaluating the concentration of fetal DNA, especially a method for evaluating the concentration of fetal DNA of a female fetus, may be established by using the method based on a single-molecule sequencing platform.

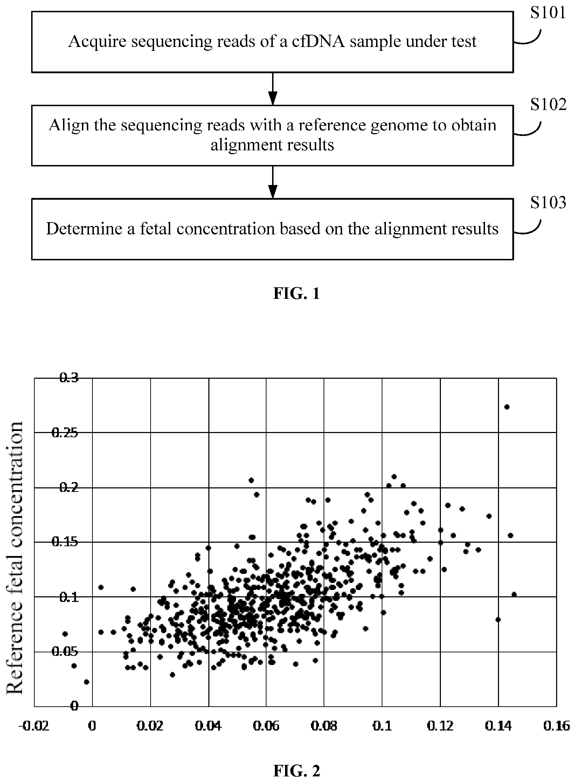

FIG. 1 is a flowchart of a method for determining a concentration of fetal DNA according to an embodiment of the present application. Referring to FIG. 1, the method may include the steps below.

In S101, multiple sequencing reads of a cfDNA sample under test are acquired.

cfDNA refers to highly fragmented cell-free DNA present in the circulating human blood. In the embodiments of the present application, the cfDNA sample under test is a cfDNA sample where the concentration of fetal DNA needs to be determined. For example, the cfDNA sample may be peripheral blood of a pregnant woman that contains free DNA.

In the embodiments of the present application, the cfDNA sample under test refers to a cfDNA-containing sample where the concentration of fetal DNA is to be determined. The sequencing reads of the cfDNA sample under test refer to reads obtained through the sequencing of cfDNA in the sample under test, that is, sequencing reads of fragmented DNA in the sample under test. It is to be understood that the sequencing reads of the cfDNA sample under test have no requirements on a sequencing instrument and a sequencing platform. That is, the sequencing reads of the cfDNA sample under test may come from sequencing data obtained by any sequencing platform. In some embodiments, the sequencing reads of the cfDNA sample under test may be obtained by a single-molecule sequencing platform (such as a GenoCare sequencing platform or a nanopore sequencing platform of GeneMind) or may be obtained by a second-generation sequencing platform (such as an Illumina platform or a BGI platform for second-generation sequencing). For example, the sequencing reads of the cfDNA sample under test are data obtained through DNA sequencing of the cfDNA sample under test based on the single-molecule sequencing platform. Therefore, by the method according to the embodiment of the present application, the sequencing reads of the cfDNA sample under test may be from the single-molecule sequencing platform so that the concentration of fetal DNA in the cfDNA sample under test can be analyzed.

In S102, the multiple sequencing reads are aligned with a reference genome to obtain alignment results.

In this step, the reference genome refers to a human reference genome, such as, but not limited to, a human reference genome hg19.

In S103, the concentration of fetal DNA is determined based on the alignment results.

In the embodiments of the present application, to accurately obtain the final concentration of fetal DNA, the sequencing reads are aligned with the reference genome. The obtained alignment results may be alignment results corresponding to probabilities of alignment of maternal and fetal cfDNA to different bins of the reference genome. The obtained alignment results may be alignment results corresponding to differences in the distribution of two ends of maternal and fetal cfDNA at nucleosome positions. The obtained alignment results may be alignment results corresponding to the probabilities of alignment of maternal and fetal cfDNA to different bins of the reference genome and the differences in the distribution of two ends of maternal and fetal cfDNA at the nucleosome positions. The obtained alignment results may be new alignment results formed after the preceding alignment results are processed.

The method for determining the concentration of fetal DNA according to the embodiment of the present application is described in detail below.

**The concentration of fetal DNA is determined according to differences in the probabilities of alignment of the maternal and fetal cfDNA to different bins of the reference genome.**

Free DNA (cfDNA) obtained from the peripheral blood of a mother comes partly from the mother and partly from a fetus. When the maternal and fetal DNA is aligned with the reference genome, coverage depths of the maternal and fetal DNA in different regions of the reference genome are different. Therefore, the concentration of fetal DNA may be determined according to the alignment results corresponding to the differences.

In an implementation of the embodiment of the present application, the concentration of fetal DNA is determined according to the differences in the probabilities of alignment of the maternal and fetal cfDNA to different bins of the reference genome. Specifically, the method for determining the concentration of fetal DNA includes the steps below.

In S111, the multiple sequencing reads of the cfDNA sample under test are acquired.

In this step, the cfDNA sample under test is a cfDNA sample where the concentration of fetal DNA needs to be determined. The method according to the embodiment of the present application has no explicit requirements on a source of the cfDNA sample under test and characteristic parameters of the cfDNA sample under test.

In S112, the multiple sequencing reads are aligned with the reference genome to obtain the alignment results.

This step includes S1121 and S1122.

In S1121, the reference genome is segmented into multiple segment bins.

In S1122, a first alignment count of sequencing reads falling within each segment bin is determined, and first alignment counts of the multiple segment bins are determined as the alignment results.

It is to be noted that "first" in the first alignment count is merely for distinguishing the first alignment count from the subsequent alignment count, and there is no order between "first" and "second". In the method, the reference genome is segmented according to a fixed length, and then the number of sequencing reads falling within each segment bin is counted. That is, how many sequencing reads fall within the corresponding segment bin is calculated. The number of sequencing reads falling within each segment bin is the first alignment count, which is an alignment result in this scenario. Correspondingly, in some scenarios, the number of sequencing reads falling within each segment bin may also be referred to as "coverage". For ease of description, in the subsequent embodiments of the present application, the number is referred to as an alignment count and is referred to as the "first alignment count" to be distinguished from an alignment result of another type.

It is to be understood that the "first alignment count" in the embodiments of the present application may be an absolute number of sequencing reads falling within the corresponding segment bin or may be a median of sequencing reads falling within the corresponding segment bin. Additionally, in the statistical process, the number of sequencing reads falling within each segment bin is counted, allowing for a certain error tolerance. That is, a sequencing read having a preset number of base errors within a certain length range is allowed to be used as an aligned sequencing read.

To accurately obtain the alignment results, the reference genome may be segmented according to the fixed length. For example, a segmentation length of the reference genome is determined according to features of the sequencing reads.

Some special chromosomes exist in the reference genome. These chromosomes may introduce a certain bias or other peculiarities into the process of determining the concentration of fetal DNA through alignment with the reference genome. In an implementation, segment bins of the special chromosomes (referred to as "particular chromosomes" below) are removed from the reference genome to reduce the bias caused by these chromosomes and minimize an effect of the peculiarities of these chromosomes on test results. In some embodiments, each particular chromosome includes at least one of chromosome X, chromosome Y, a mitochondrial chromosome, chromosome 13, chromosome 18, or chromosome 21. Segment bins corresponding to chromosome X and chromosome Y are removed so that differences of a male fetus and a female fetus in chromosome X and chromosome Y can be prevented from affecting results of the subsequent concentration quantitation model. Chromosome 13, chromosome 18, and chromosome 21 are removed due to relatively large probabilities of duplications and deletions of these chromosomes. One screening goal of NIPT is to screen the duplications and deletions of chromosome 13, chromosome 18, and chromosome 21. When chromosome 13, chromosome 18, and chromosome 21 are used as reference chromosomes, deviations may appear in data normalization statistics, for example, normalized alignment counts are affected, affecting calculation results and the accuracy of NIPT screening results.

In the embodiments of the present application, particular chromosomes may be adjusted according to expected requirements of detection of the concentration of fetal DNA. For example, chromosome X and chromosome Y are removed, chromosome 13, chromosome 18, and chromosome 21 are removed, or the mitochondrial chromosome is removed. Of course, multiple removals of the preceding removals may be performed simultaneously. For example, chromosome X, chromosome Y, the mitochondrial chromosome, chromosome 13, chromosome 18, and chromosome 21 or the corresponding segment bins are all removed so that the segment bins into which the reference genome is segmented do not include the segment bins corresponding to the preceding chromosomes.

In the present application, the particular chromosomes may be excluded from the segment bins in various manners. The segment bins obtained through segmentation may be screened based on the particular chromosomes. Alternatively, the particular chromosomes may be removed before segmentation. Specifically, the corresponding processing manner may be selected based on practical application requirements and is not limited in the present application.

In an embodiment, that the reference genome is segmented into the multiple segment bins includes: based on a preset segmentation length, segmenting the reference genome into multiple initial segment bins; and removing segment bins corresponding to the particular chromosome from the multiple initial segment bins to obtain the multiple segment bins. That is, after the reference genome is segmented into the multiple segment bins, the segment bins corresponding to the particular chromosome in the reference genome are removed and excluded from calculation. It is to be understood that the "segment bins corresponding to the particular chromosome" refer to multiple segment bins formed after the particular chromosome is segmented based on the preset segmentation length.

In another embodiment, that the reference genome is segmented into the multiple segment bins includes: removing the particular chromosome from the reference genome to obtain a removed reference genome; and based on the preset segmentation length, segmenting the removed reference genome into the multiple segment bins. That is, the particular chromosome is removed before the reference genome is segmented.

The preset segmentation length may be adjusted according to a data volume of data processing and an expected relative accuracy. For example, the reference genome may be segmented according to a length of every 50,000 bases. To make the obtained first alignment count more accurate, the first alignment count may be corrected to obtain a corrected first alignment count so that corrected first alignment counts are determined as the alignment results.

In some embodiments, correction includes GC correction. The GC correction is an operation in a bioinformatics analysis process. The GC correction is performed because gene sequencers and the corresponding sequencing processes have certain GC biases. For example, some sequencers have a higher probability of detecting high-GC reads, while some sequencers have a higher probability of detecting low-GC reads. In this embodiment, the concentration of fetal DNA is determined based on alignment probabilities in different regions of the reference genome. A GC bias of a sequencer affects the determination of actual alignment probabilities in different regions. Therefore, the GC correction is required to improve accuracy.

Correcting the first alignment count to obtain the corrected first alignment count includes: based on the first alignment count of each segment bin and a mean of the first alignment counts of the multiple segment bins, normalizing the first alignment count of each segment bin to obtain a normalized first alignment count of each segment bin; and performing the GC correction on the normalized first alignment count of each segment bin to obtain a GC-corrected first alignment count. The mean of the first alignment counts of the multiple segment bins refers to the ratio of a sum of the first alignment counts of the segment bins to the total number of the segment bins.

After the first alignment counts of the multiple segment bins are obtained, to facilitate calculation and processing, the first alignment counts of the multiple segment bins are normalized by the corresponding formula:

the normalized first alignment count of each segment bin = the first alignment count of each segment bin/the mean of the first alignment counts of the multiple segment bins.

After the first alignment count of each segment bin is obtained, the GC correction is performed to obtain the GC-corrected first alignment count.

In an embodiment, performing the GC correction on the normalized first alignment count of each segment bin to obtain the GC-corrected first alignment count includes the steps below.

(1) A first relationship curve is generated based on a GC content and the normalized first alignment count of each segment bin.

In this embodiment, the GC content may be represented by various parameters, such as the GC content or a GC content median of each segment bin, or may be represented by other parameters that can reflect the GC content of the segment bin.

In the embodiments of the present application, GC filtering may be performed on the segment bins forming the first relationship curve. For example, the segment bins are filtered according to GC contents so that some abnormal or statistically insignificant segment bins are filtered out, thereby optimizing the first relationship curve and reducing data processing resources occupied during data processing.

In an embodiment, the GC filtering is implemented in the manner below. Before the first relationship curve is generated based on the GC content and the normalized first alignment count of each segment bin, the following processing is performed: each segment bin is filtered based on the GC content, and each segment bin whose GC content does not satisfy a preset requirement is removed so that the first relationship curve is generated based on the GC content of each segment bin after GC filtering and the normalized first alignment count of each segment bin.

In another embodiment, the GC filtering is implemented in the manner below. In the process of, based on the first relationship curve, performing the GC correction on the normalized first alignment count, each segment bin is filtered based on the GC content, and based on the first relationship curve, the GC correction is performed on the normalized first alignment count of each segment bin after GC filtering to obtain the GC-corrected first alignment count.

In the preceding embodiments, as an example, the first relationship curve is generated based on the GC content and the normalized first alignment count of each segment bin in the manner below. An x-axis represents the GC content of each segment bin (that is, a proportion of GC bases corresponding to the segment bin of the reference genome), and a y-axis represents the normalized first alignment count of each segment bin. The segment bins and data on GC contents of the segment bins are integrated. That is, a scatter plot is generated according to the GC content and the normalized first alignment count of each segment bin. Scatter data in the scatter plot are smoothed to obtain a smooth curve. The scatter data in the scatter plot may be smoothed using an algorithm such as locally weighted scatterplot smoothing (LOWESS) to obtain the smooth curve, which is the first relationship curve, for example, $y_s = f(x_s)$.

Specifically, as an example, the first relationship curve may be generated in the manner below. The abscissa is divided into different sections, a GC content median of points in the same section is calculated, the GC content median represents a value corresponding to the section, and all GC content medians are plotted and smoothed to obtain the first relationship curve. In another example, all data may be directly smoothed. That is, all data points are considered and smoothed to obtain the smooth curve. Thus, a Spearman correlation coefficient obtained through a test set is higher. It is to be noted that this smoothing method is also applicable to the subsequent alignment probability smoothing and correction.

(2) Based on the first relationship curve, the GC correction is performed on the normalized first alignment count to obtain the GC-corrected first alignment count.

In some embodiments, the GC correction is performed according to a subtraction or division relation between the normalized first alignment count of each segment bin and the GC content of each segment bin to determine the GC-corrected first alignment count.

In an example, the GC correction method is "subtraction", and the corresponding correction formula is $y_{alignment\ probability} = y - f(x)$. In another example, the GC correction method is "division", and the corresponding correction formula is $y_{alignment\ probability} = y/f(x)$.

In the above two correction formulas, x represents the GC content of the segment bin, y represents the normalized first alignment count of the segment bin, and $y_{alignment\ probability}$ represents the GC-corrected normalized first alignment count.

In some embodiments, the correction further includes alignment probability correction to improve the accuracy of the obtained first alignment count. That is, in some embodiments, the correction includes both the GC correction and the alignment probability correction.

In an implementation of the embodiment of the present application, correcting the first alignment count to obtain the corrected first alignment count further includes: cutting the reference genome based on a particular sliding window length to obtain sequence cuts, aligning the sequence cuts with the reference genome, counting a first alignment count of sequence cuts falling within each segment bin, and determining the first alignment count as a sliding window alignment count of each segment bin; based on the sliding window alignment count, determining a normalized sliding window alignment count of each segment bin; and performing the alignment probability correction based on the normalized sliding window alignment count and the normalized first alignment count of each segment bin to determine a first alignment count subjected to the alignment probability correction.

In the embodiments of the present application, the first alignment count is corrected by the "alignment probability correction" method based on the following consideration: if a different region of a reference sequence of the reference genome is cut according to a particular length as a sequencing read and then aligned with the reference genome, the sequencing read is aligned to different segment bins for different times. Since sequences in some regions are similar to those at multiple positions of reference sequences of the reference genome, these regions are more easily aligned. For example, reference sequences of the human genome hg19 are cut according to a step size of 2 bases and a sliding window of 37 bases to obtain sequence cuts, and these sequence cuts are aligned with the human reference genome hg19. An alignment count of sequence cuts in a different segment bin of the human reference genome is counted, which may be referred to as the "sliding window alignment count". Specifically, the particular sliding window length may be considered based on a practical application scenario. For example, the particular sliding window length is determined based on the characteristics of the reference genome or the characteristics of the sample under test. After the particular sliding window length is determined, the reference genome is cut, the sequence cuts are aligned with the reference genome, the first alignment count of sequence cuts falling within each segment bin is counted, and the first alignment count is determined as the sliding window alignment count. After the sliding window alignment count is obtained, to facilitate calculation and processing, the normalized sliding window alignment count of each segment bin is determined based on the sliding window alignment count; and the alignment probability correction is performed on the normalized sliding window alignment count. For the alignment probability correction method, refer to the preceding GC correction method.

In an embodiment, performing the alignment probability correction based on the normalized sliding window alignment count and the normalized first alignment count of each segment bin to determine the first alignment count subjected to the alignment probability correction includes the steps below.

(1) A second relationship curve is generated based on the normalized sliding window alignment count of each segment bin and the normalized first alignment count corresponding to each segment bin.

The normalized sliding window alignment count of each segment bin may be represented by various parameters, such as the normalized sliding window alignment count or a median of sliding window alignment counts of each segment bin, or may be represented by other parameters that can reflect the normalized sliding window alignment count of each segment bin.

In the embodiments of the present application, normalized sliding window alignment counts forming the second relationship curve may be filtered so that some abnormal or statistically insignificant segment bins are filtered out, thereby optimizing the second relationship curve and improving the accuracy of related data processing based on the second relationship curve.

In an embodiment, the normalized sliding window alignment counts forming the second relationship curve are filtered in the manner below. Before the second relationship curve is generated based on the normalized sliding window alignment count of each segment bin and the normalized first alignment count corresponding to each segment bin, the following processing is performed: the segment bins are filtered based on the normalized sliding window alignment counts, and segment bins whose normalized sliding window alignment counts are each not less than a first target threshold are obtained so that the second relationship curve is generated for the segment bins whose normalized sliding window alignment counts are each not less than the first target threshold.

In an embodiment, the normalized sliding window alignment counts forming the second relationship curve are filtered in the manner below. In the process of, based on the second relationship curve, performing the alignment probability correction on the normalized first alignment count to obtain the first alignment count subjected to the alignment probability correction, the segment bins are filtered based on the normalized sliding window alignment counts, and second relationship curve sections of the second relationship curve where normalized sliding window alignment counts are each not less than the first target threshold are retained to obtain a second relationship curve after alignment probability filtering; and based on the second relationship curve after alignment probability filtering, the alignment probability correction is performed on the normalized sliding window alignment count of each segment bin to obtain the first alignment count subjected to the alignment probability correction.

In the preceding embodiments, based on repeated verification, the first target threshold may be 0.8. That is, each segment bin whose normalized sliding window alignment count is less than 0.8 is removed.

In the preceding embodiments, as an example, the second relationship curve is generated based on the normalized sliding window alignment count of each segment bin and the normalized first alignment count corresponding to each segment bin in the manner below. An x-axis represents the normalized sliding window alignment count of each segment bin, and a y-axis represents the normalized first alignment count corresponding to each segment bin. The segment bins and data on the GC contents of the segment bins are integrated. That is, a scatter plot is generated according to the GC content of each segment bin and the normalized first alignment count corresponding to each segment bin. Scatter data in the scatter plot are smoothed to obtain a smooth curve. For example, the scatter data in the scatter plot may be smoothed using the LOWESS algorithm.

(2) Based on the second relationship curve, the alignment probability correction is performed on the normalized first alignment count to obtain the first alignment count subjected to the alignment probability correction.

In some embodiments, the alignment probability correction is performed according to a subtraction or division relation between the normalized first alignment count of each segment bin and the sliding window alignment count of each segment bin to determine the first alignment count subjected to the alignment probability correction.

In an example, the alignment probability correction method is "subtraction", and the corresponding correction

formula is $y_{\text{alignment probability}} = y - f(x)$. In another example, the alignment probability correction method is "division", and the corresponding correction formula is $y_{\text{alignment probability}} = y/f(x)$.

In the above two correction formulas, x represents the normalized sliding window alignment count of each segment bin, y represents the normalized first alignment count of each segment bin, and $y_{\text{alignment probability}}$ represents the normalized first alignment count subjected to the alignment probability correction.

In S113, the concentration of fetal DNA is determined based on the alignment results.

In the embodiments of the present application, the concentration of fetal DNA may be determined by a machine learning method in conjunction with a neural network. In an implementation of the embodiment of the present application, that the concentration of fetal DNA is determined based on the alignment results includes the steps below.

First training sample data are obtained, where each sample in the first training sample data is labeled with first feature values and a first target value, the first feature values are sample alignment counts, and the first target value is an actual concentration of fetal DNA.

Based on a particular model structure, machine learning modeling is performed on the first training sample data to obtain a first concentration quantitation model of fetal DNA.

The alignment results are input into the first concentration quantitation model of fetal DNA to obtain a first concentration of fetal DNA and the first concentration of fetal DNA is determined as the concentration of fetal DNA.

In some embodiments, based on the particular model structure, performing the machine learning modeling on the first training sample data to obtain the first concentration quantitation model of fetal DNA includes the steps below.

The first training sample data are divided into a training set and a test set. The machine learning modeling is performed based on the training set to obtain an initial model.

The test set is processed based on the initial model to obtain a predicted concentration of fetal DNA in each test sample in the test set.

The predicted concentration of fetal DNA is compared with an actual concentration of fetal DNA in each test sample in the test set to obtain a comparison result.

A model parameter of the initial model is adjusted based on the comparison result to obtain the first concentration quantitation model of fetal DNA.

The first training sample data may be data in a database of cfDNA in peripheral blood of pregnant women and with known concentrations of fetal DNA, where the data are obtained based on various sequencing platforms including the single-molecule sequencing platform. For example, a certain number of samples with known concentrations of fetal DNA are used, the reference sequences of chromosomes of the reference genome are segmented into bins (for example, bins of 5,000 bases, 50,000 bases, 100,000 bases, 300,000 bases, or 800,000 bases, for example), and alignment counts of alignment data of each sample in different segment bins are separately counted. The alignment counts are normalized and subjected to the GC correction and the alignment probability correction by the LOWESS method, the corrected normalized alignment counts of the different segment bins are used as the feature values, and the known actual concentration of fetal DNA in each sample is used as the target value, and a particular model is used for learning and modeling. The particular model may be a classification model, a convolutional neural network model, or the like. The particular model structure is not limited in the embodiments of the present application as long as the training sample data can be learned and the concentration of fetal DNA can be predicted. A model learning and training process is an iterative process. Therefore, training samples may be divided into the training set and the test set. The accuracy of the currently trained model is tested by the test set. If the accuracy is lower than a set accuracy threshold, the model parameter in the model structure of the current model is adjusted by using the test set until an error between an output concentration of fetal DNA of the obtained model and the actual concentration of fetal DNA satisfies a corresponding error range.

In the case of a relatively small number of samples for machine learning and relatively many feature values (for example, about three thousand to six hundred thousand features, depending on a size of the segment bin of the reference sequences of the chromosomes), overfitting cannot be avoided in a dimensionality reduction or learning process. In the finally built concentration quantitation model of fetal DNA, a prediction effect of the concentration of fetal DNA in the test set has a certain deviation from the actual concentration of fetal DNA. For example, in the concentration quantitation model of fetal DNA built using 615 samples with known concentrations of fetal DNA, $R^2$ corresponding to the test set of the model is only about 0.3, and a Pearson correlation coefficient between actual values and predicted values is about 0.5. However, when the alignment counts are normalized and corrected (the GC correction or a combination of the GC correction and the alignment probability correction) by the preceding method according to the embodiment of the present application, the correlation coefficient between the predicted values and the actual values of the test set increases. As can be known from the comparison between the modeling directly performed by using the normalized alignment counts of the segment bins and the modeling performed by using the normalized alignment counts of the segment bins subjected to the GC correction and the alignment probability correction, the modeling after normalization, the GC correction, and the alignment probability correction has a better effect, that is, the correlation coefficient between the predicted values and the actual values of the test set is higher.

In another embodiment, that the concentration of fetal DNA is determined based on the alignment results includes the steps below.

The alignment results are input into a first preset model to obtain an initial concentration of fetal DNA.

The initial concentration of fetal DNA is corrected based on a second preset model to obtain the concentration of fetal DNA.

The first preset model is a linear relationship model built based on alignment results in second sample data and an initial concentration of fetal DNA corresponding to the alignment results, and the second sample data include the alignment results of alignment of sequencing reads of a cfDNA sample with the reference genome and the concentration of fetal DNA corresponding to the alignment results. The second preset model is a model obtained after the first preset model is processed based on constants determined through linear fitting.

For example, the first preset model may be $FF1 = (\overrightarrow{data})$, where $\overrightarrow{data}$ represents the alignment results, and FF1 represents the initial concentration of fetal DNA. The second preset model may be $FF2 = FF1 \times c + d$, where c and d represent the constants determined through linear fitting, and FF2 represents the concentration of fetal DNA.

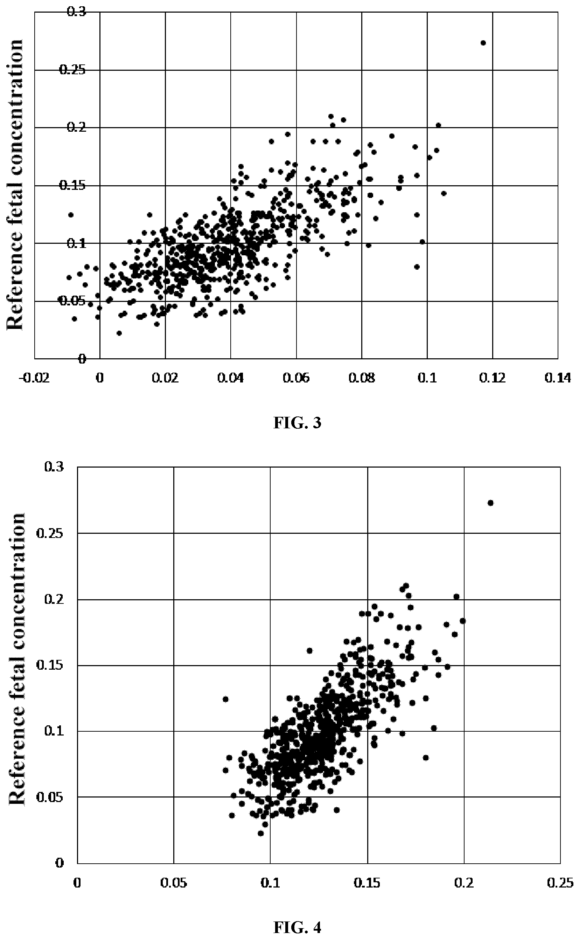

For example, SeqFF is used for model building and estimation of the concentration of fetal DNA according to differences in alignment probabilities of samples in different bins of the reference sequences of the reference genome. The SeqFF method uses two machine learning models, one is WRSC and the other is Enet. The final output concentration of fetal DNA is a mean of predicted values of the two models. When the SeqFF model is tested using data from the sequencing platforms, especially the single-molecule sequencing platform, the WRSC model has a better effect than Enet. That is, a linear correlation coefficient between concentrations of fetal DNA estimated by WRSC and actual concentrations of fetal DNA is higher than that of Enet or SeqFF. Therefore, a model parameter involved in the WRSC algorithm is preferentially considered. Specifically, reference may be made to FIGS. 2 and 3. FIG. 2 is a schematic diagram of a relationship between concentrations of fetal DNA calculated by an Enet model and reference concentrations of fetal DNA according to an embodiment of the present application. FIG. 3 is a schematic diagram of a relationship between concentrations of fetal DNA calculated by a WRSC model and reference concentrations of fetal DNA according to an embodiment of the present application. As can be seen from the figures, a positive correlation between the concentrations of fetal DNA calculated by the WRSC model and the reference concentrations of fetal DNA is superior to a positive correlation between the concentrations of fetal DNA calculated by the Enet model and the reference concentrations of fetal DNA.

For example, in the WRSC model, the reference sequences of the chromosome are segmented according to a bin of a particular number of bases, such as 50,000 bases. After training using the WRSC algorithm, a linear relationship between coverage densities in different bins of the reference sequences and the concentration of fetal DNA is found, as shown by the following formula. In the following formula, FF represents the concentration of fetal DNA, $\overrightarrow{data}$ represents the coverage densities in different bins of the reference sequences, $\vec{A}$ represents a constant vector whose dimensions are equal to those of $\overrightarrow{data}$, B represents a parameter matrix for linear transformation, and c and d represent constants:

$$FF = \mathrm{sum}\{(\overrightarrow{data} - \vec{A}) \times B\} \times c + d$$

SeqFF is a model trained based on data of second-generation sequencing whose sequencing principle has a relatively large difference from that of single-molecule sequencing. Therefore, if the preceding model parameters of SeqFF are directly used to calculate the concentration of fetal DNA based on data obtained by other sequencing platforms including the single-molecule sequencing platform, the calculated concentration of fetal DNA has a relatively large difference from the actual concentration of fetal DNA. 615 NIPT clinical samples with the known concentrations of fetal DNA in the preceding embodiment are still used as examples for analysis and testing. Data in original alignment files (such as Sequence Alignment/Map (SAM) files) from the other sequencing platforms including the single-molecule sequencing platform are directly processed by using the algorithm and parameters of SeqFF. The Pearson correlation coefficient between the obtained intermediate parameters and the actual concentrations of fetal DNA is 0.714.

A linear relationship between calculated values of the concentrations of fetal DNA calculated by the SeqFF model and the actual concentrations of fetal DNA is relatively good. Therefore, the model parameters included in sum{($\overrightarrow{data}$-$\vec{A}$)×B} in the above formula may be used to calculate an intermediate parameter of the concentration of fetal DNA. Then, values of c and d are self-fitted in a linear regression manner so that model parameters applicable to the current other sequencing platforms including the single-molecule sequencing platform are obtained.

615 NIPT clinical samples with the known concentrations of fetal DNA in the preceding embodiment are still used as examples for analysis and testing. Instead, the first alignment count of sequencing reads falling within each segment bin is used for analysis; regions of chromosomes X, Y, M, 13, 18, and 21 and the mitochondrial chromosome of the reference genome where alignment probabilities are abnormal are removed for analysis; the correction method is optimized (the GC correction or a combination of the GC correction and the alignment probability correction). The data are re-analyzed by using the optimized parameters. The Pearson correlation coefficient between the intermediate parameters and the concentrations of fetal DNA is 0.755. The test set (30%) and the training set (70%) are randomly divided 100 times to establish a linear model and determine the above constant parameters c and d. An average prediction $R^2$ value corresponding to the test set and obtained from 100 times of modeling is 0.561.

It is to be noted that when the linear model is truly built, the test set and the training set are divided to evaluate, by limited data, the reliability of the model currently built based on the training set randomly divided. Therefore, the test set (30%) and the training set (70%) are randomly divided 100 times. The average prediction $R^2$ value corresponding to the test set and obtained from 100 times of modeling is 0.561.

Nine examples in which the concentration of fetal DNA is determined according to the differences in the probabilities of alignment of the maternal and fetal cfDNA to different bins of the reference genome are shown below in Table 1.

In Table 1, Example 1 uses the original conditions in the seqFF WRSC method. In the table, "Bin" in "Bin Filtering" refers to the segment bin on the reference sequences of the genome, "Mappability Correction" refers to the "alignment probability correction", "Mappability" refers to the normalized sliding window alignment count, and "groupby" refers to a GC correction method in the seqFF method. Groupby is equivalent to the following: when LOWESS smoothing is performed, bins are grouped into sections according to the GC contents, the GC content of each section is represented by a GC content value retained to three decimal places, a median of normalized counts of all segment bins corresponding to the GC content is used as the normalized count for the GC content. As mentioned in the table, such a processing manner is labeled as "median". In contrast, a normalization manner labeled as "all" is also included in the table. "All" refers to the consideration of the GC contents and the normalized alignment counts of all the segment bins during the LOWESS smoothing, without grouping the bins according to the GC contents. That is, the data of all the segment bins are used during smoothing. "Median" refers to the median. "Frac" refers to a parameter used for the LOWESS smoothing. "All mapped" refers to all aligned reads. "Unique" refers to uniquely aligned reads (that is, reads aligned to only one bin on the chromosomes). "XYM" or "chromosomes XYM" refer to chromosome X, chromosome Y, and the mitochondrial chromosome. "Flag filtering" refers to the filtering of different segment bins as described in an original seqFF paper, with a filtering file provided. "SeqFF bin flag filtering" refers to the filtering according to flags marked in the original paper. However, the finally selected solution does not use the filtering according to the flags in the original paper.

Table 1

| Example | Smoothing Method | Correction Method | Correction Content | Read Data | Bin Filtering | Pearson Correlation Coefficient (615 Clinical Samples) |
|---|---|---|---|---|---|---|
| 1 | LOWESS. Retain the GC value to three decimal places. Groupby: select the median as the count corresponding to each GC value (the normalized count corresponding to the section) and then perform LOWESS. Frac = 0.3. Abbreviation: median/-LOWESS.Frac = 0.3. | Calculate a median of the normalized counts of all the bins, calculate a difference between a LOWESS result corresponding to each GC value and the median, and subtract the difference corresponding to each GC value (three decimal places) of each bin from the normalized count of each bin. Abbreviation: subtraction. | GC | All mapped | Original seqFF filtering manner: removal of chromosomes XYM and SeqFF bin flag filtering. | 0.71425 |

(continued)

| Example | Smoothing Method | Correction Method | Correction Content | Read Data | Bin Filtering | Pearson Correlation Coefficient (615 Clinical Samples) |
|---|---|---|---|---|---|---|
| 2 | Median/LOWESS.Frac = 0.3 | Subtraction | GC | Uniqu e | Original seqFF filtering man-ner: removion of chromosomes XYM and SeqFF bin flag filtering. | 0.74890 |
| 3 | Directly use the GC or mappability (retained to 5 decimal places) of all the bins as x, use the normalized counts of all the bins as y, and perform LOWESS without groupby or calculating the median. Frac = 0.2. Abbreviation: all/LO-WESS.Frac = 0.2. | Divide the nor-malized count of each bin by the LOWESS result correspondi ng to the GC value of the bin. Abbreviatio n: Division. | GC/Mappabili ty | Uniqu e | Filtering according to GM-hg19-50kbin-bed data: re-moval of chro-mosomes XYM, 13, 18, and 21, re-moval of N re-gions, and re-moval of each bin whose mappability is less than 0.8. | 0.74432 |
| 4 | All/LOWESSS.Frac = 0.2 | Subtraction | GC/Mappabili ty | Uniqu e | Filtering according to GM-hg19-50kbin-bed data: re-moval of chro-mosomes XYM, 13, 18, and 21, re-moval of N re-gions, and re-moval of each bin whose mappability is less than 0.8. | 0.74437 |

(continued)

| Example | Smoothing Method | Correction Method | Correction Content | Read Data | Bin Filtering | Pearson Correlation Coefficient (615 Clinical Samples) |
|---------|------------------|-------------------|--------------------|-----------|---------------|-------------------------------------------------------|
| 5 | Median/LOWESSS.Frac = 0.25 | Subtraction | GC/Mappability | Uniqu e | Filtering according to GM-hg19-50kbinbed data: removal of chromosomes XYM, 13, 18, and 21, removal of N regions, and removal of each bin whose mappability is less than 0.8. | 0.74382 |
| 6 | All/LOWESS.Frac = 0.2 | Division | GC/Mappability | Uniqu e | Filtering according to GM-hg19-50kbinbed data: removal of chromosomes XYM, 13, 18, and 21, removal of N regions, and no filtering according to the mappability. | 0.75068 |
| 7 | Median/LOWESSS.Frac = 0.25 | Subtraction | GC/Mappability | Uniqu e | Filtering according to GM-hg19-50kbinbed data: removal of chromosomes XYM, 13, 18, and 21, removal of N regions, and no filtering according to the mappability. | 0.74998 |

(continued)

| Example | Smoothing Method | Correction Method | Correction Content | Read Data | Bin Filtering | Pearson Correlation Coefficient (615 Clinical Samples) |
|---|---|---|---|---|---|---|
| 8 | All/LOWESS.Frac = 0.2 | Subtraction | GC | Uniqu e | Filtering according to GM-hg19-50kbin-bed data: removal of chromosomes XYM, 13, 18, and 21, removal of N regions, and no filtering according to the mappability. | 0.75471 |
| 9 | Median/LOWESSS.Frac = 0.25 | Subtraction | GC | Uniqu e | Filtering according to GM-hg19-50kbin-bed data: removal of chromosomes XYM, 13, 18, and 21, removal of N regions, and no filtering according to the mappability. | 0.75457 |

The Pearson correlation coefficient evaluates a linear correlation between the values directly calculated by the algorithm and the reference concentrations of fetal DNA. Referring to FIG. 4, although the linear correlation between the calculated values and the reference concentrations of fetal DNA is high, great differences exist between absolute values of the calculated values and the reference concentrations of fetal DNA. Therefore, a linear model is needed to correct the calculation results of the algorithm, to accurately estimate the concentration of fetal DNA. Fitting correction may be performed by using the preceding formula $FF = \text{sum}\{(\overrightarrow{data\text{-}A}) \times B\} \times c + d$. In the formula, $\text{sum}\{(\overrightarrow{data\text{-}A}) \times B\}$ represents numerical results calculated by gmFF_V7, and c and d represent two parameters fitted through the linear fitting and are used as constants in the subsequent model.

In Table 1, different processing parameters and conditions are evaluated according to the Pearson correlation coefficient between the predicted concentrations of fetal DNA and the reference concentrations of fetal DNA (concentrations of fetal DNA provided by another platform).

As can be seen from Table 1, compared with the algorithm (whose Pearson correlation coefficient is 0.71425) in Example 1, the other algorithms have Pearson correlation coefficients all greater than 0.74, and the algorithms in Example 6, Example 8, and Example 9 have Pearson correlation coefficients greater than 0.75. Compared with the other algorithms, the algorithm in Example 8 can obtain the highest Pearson correlation coefficient of 0.75471.

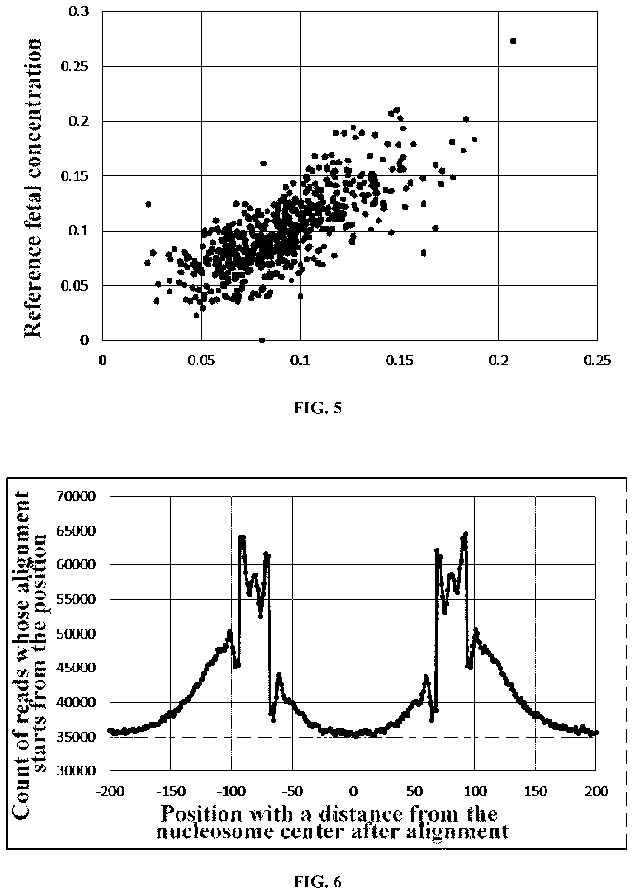

After correction, a relationship between the calculated concentrations of fetal DNA and the reference concentrations of fetal DNA in Example 8 is shown in FIG. 5. As can be seen from FIG. 5, there is a high positive correlation between the calculated concentrations of fetal DNA and the reference concentrations of fetal DNA.

**The concentration of fetal DNA may be calculated by using the differences in the distribution of two ends of the maternal and fetal cfDNA at the nucleosome positions.**

A nucleosome is the fundamental unit of human chromatin. When DNA is subjected to enzymatic cleavage, DNA is more difficult to cut within the nucleosome than in a linker region between nucleosomes. Therefore, during digestion, DNAis more likely to be cleaved in the linker region between nucleosomes. Due to differences in the structural characteristics of nucleosomes in the maternal and fetal DNA during apoptosis, the binding strength of the nucleosomes also differs. Therefore, during digestion, the probabilities of cleavage of the maternal and fetal DNA within the nucleosome and in the linker region between nucleosomes are different. Specifically, the fetal DNA is cleaved within the nucleosome at a higher probability than the maternal DNA. Therefore, the distribution of ends of cfDNA in the cfDNA sample under test at the nucleosome positions is evaluated so that the concentration of fetal DNA can be obtained.

Another embodiment of the present application provides a method for determining the concentration of fetal DNA by using the differences in the distribution of two ends of the maternal and fetal cfDNA at the nucleosome positions. Specifically, the method for determining the concentration of fetal DNA includes the steps below.

In S 121, the multiple sequencing reads of the cfDNA sample under test are acquired.

In this step, the cfDNA sample under test is a cfDNA sample where the concentration of fetal DNA needs to be determined. The method according to the embodiment of the present application has no explicit requirements on a source of the cfDNA sample under test and characteristic parameters of the cfDNA sample under test.

In S 122, the multiple sequencing reads are aligned with the reference genome to obtain the alignment results.

This step includes S1221 to S1224.

In S1221, a second alignment count of times each base site of the reference genome acts as starting positions of alignment of sequencing reads with the reference genome is determined, and based on the second alignment count, a nucleosome center score corresponding to each base site of the reference genome is calculated.

In S1222, nucleosome center positions are determined based on nucleosome center scores corresponding to base sites of the reference genome and a center score screening threshold.

In S1223, based on the nucleosome center positions, alignment and summation are performed on second alignment counts at corresponding positions within all nucleosome regions to obtain summed second alignment counts.

In S1224, dimensionality reduction is performed on the summed second alignment counts to obtain normalized second alignment counts subjected to the dimensionality reduction, and the normalized second alignment counts subjected to the dimensionality reduction are determined as the alignment results.

It is to be noted that the second alignment count in this embodiment is intended to be distinguished from the first alignment count in the preceding embodiments, and there is no order between the first alignment count and the second alignment count. A count of times each base site of the reference genome acts as the starting positions of alignment of the sequencing reads with the reference genome is determined as the second alignment count. Correspondingly, the second alignment count may also be referred to as the "count". That is, starting positions of alignment of different reads are counted, and then a count of times a different site of the reference genome is just starting positions of alignment of reads are just is obtained.

Since a cfDNA incision (that is, a starting end of a sequencing read) is within the nucleosome at a smaller probability than in a nucleosome linker region, a range of a nucleosome region needs to be searched for and determined.

In step S1221, determining the count of times a base site of the reference genome is at starting positions of alignment of sequencing reads refers to analyzing the starting positions of alignment of all the sequencing reads and counting the number of times each site of the reference genome acts as the starting positions of alignment of these reads. In the embodiment of the present application, the count is determined as the second alignment count. It is to be understood that the second alignment count may be a counted absolute number of times each site of the reference genome acts as the starting positions of alignment of these reads or may be a median of the counted numbers of times each site of the reference genome acts as the starting positions of alignment of these reads. Additionally, in the statistical process, the number of times each site of the reference genome acts as the starting positions of alignment of these reads is counted, allowing for a certain error tolerance. That is, a sequencing read having a preset number of base errors within a certain length range is allowed to be used as an aligned sequencing read.

After the count is determined as the second alignment count, the nucleosome center score corresponding to each base site of the reference genome is calculated based on the second alignment count. The "nucleosome center score" may be understood as a score of a probability of each site being a nucleosome center position. The "nucleosome center position" refers to that the site is at the very center of the nucleosome region.

In some embodiments, calculating the nucleosome center score corresponding to each base site of the reference genome includes the steps below.

(1) A first count mean of second alignment counts of a first particular number of bases on the left and the first particular number of bases on the right of each base site of the reference genome is calculated.

(2) A second count mean of second alignment counts of a second particular number of bases on the left and the second particular number of bases on the right of each base site of the reference genome is calculated.

(3) The nucleosome center score corresponding to each base site is determined according to the first count mean and the second count mean.

The first particular number and the second particular number are determined based on the number of bases in the nucleosome region and the number of bases in the nucleosome linker region. With *x* as one base site, the first particular number = (the number of bases in the nucleosome region - 1)/2, and the second particular number = (the number of bases in the nucleosome region - 1)/2 + the number of bases in the nucleosome linker region/2.

For example, as reported in the literature, the length of the nucleosome region is 147 bases, and the linker region includes about 20 bases at one end and about 20 bases at the other end of the nucleosome. The first particular number = (147 - 1)/2 = 73, and the second particular number = (147 - 1)/2 + 20 = 93. That is, assuming that *x* represents the nucleosome center position, 73 bases on the left of the nucleosome center position and 73 bases on the right of the nucleosome center position form the nucleosome region, and the 74th base to the 93rd base on the left of the nucleosome center position and the 74th base to the 93rd base on the right of the nucleosome center position form the nucleosome linker region.

By the above formula, the ratio of a sum of a count mean in a bin of the 74th to 93rd bases on the left of a to-be-observed position and a count mean in a bin of the 74th to 93rd bases on the right of the to-be-observed position to a count mean in a bin of 73 bases on the left and 73 bases on the right of the to-be-observed position is calculated. Apparently, in the proximity of a particular nucleosome region, the closer the to-be-observed position to the nucleosome center position, the larger the nucleosome center score.

Further, the nucleosome center score corresponding to each site is determined according to the first count mean and the second count mean. The calculation formula of the nucleosome center score may be expressed as follows:

$$\text{the nucleosome center score} =$$

$$\frac{\text{count mean in a bin } [x-93, x-74] + \text{count mean in a bin } [x+93, x+74]}{\text{count mean in a bin } [x-73, x+73]}.$$

In the formula, *x* represents the base site, [x-93, x-74] represents a bin from a site 93 nucleotides away from *x* on a side of *x* to a site 74 nucleotides away from *x* on the same side, [x+93, x+74] represents a bin from a site 93 nucleotides away from *x* on the other side of *x* to a site 74 nucleotides away from *x* on the same side, and [x-73, x+73] represents a bin from a site 73 nucleotides away from *x* on the side of *x* to a site 73 nucleotides away from *x* on the other side of x.

In some embodiments, considering that a sequencing error rate of a single-molecule sequencer tends to be higher than an error rate of the second-generation sequencing, when the probability of each base in the nucleosome region being the nucleosome center position is analyzed and compared, regions with relatively high error rates at two ends of a sequence are cut off so that the accuracy and efficiency of determination of the nucleosome center position can be improved.

In some embodiments, the "nucleosome center score" and the subsequent "nucleosome ratio" are calculated after the left and right ends of the nucleosome region are each truncated by n bases, where n is a natural number less than or equal to 5. For example, n is 1, 2, 3, 4, or 5.

In this case, the calculation formula of the nucleosome center score may be expressed as follows:

$$\text{the nucleosome center score} =$$

$$\frac{count\ mean\ in\ a\ bin[x-93, x-74+n] + count\ mean\ in\ a\ bin\ [x+93, x+74-n]}{count\ mean\ in\ a\ bin[x-73+n, x+73-n]}.$$

In the formula, *x* represents the base site, [x-93, x-74+n] represents a bin from a site 93 nucleotides away from *x* on a side of *x* to a site (74-n) nucleotides away from *x* on the same side, [x+93, x+74-n] represents a bin from a site 93 nucleotides away from *x* on the other side of *x* to a site (74-n) nucleotides away from *x* on the same side, and [x-73+n, x+73-n] represents a bin from a site (73-n) nucleotides away from *x* on the side of *x* to a site (73-n) nucleotides away from *x* on the other side of *x*.

For example, when n = 5, the calculation formula of the nucleosome center score may be expressed as follows:

the nucleosome center score =

$$\frac{\text{count mean in a bin } [x-93, x-69] + \text{count mean in a bin } [x+93, x+69]}{\text{count mean in a bin } [x-68, x+68]}$$

In the formula, $x$ represents the base site, [$x$-93, $x$-69] represents a bin from a site 93 nucleotides away from $x$ on a side of $x$ to a site 69 nucleotides away from $x$ on the same side, [$x$+93, $x$+69] represents a bin from a site 93 nucleotides away from $x$ on the other side of $x$ to a site 69 nucleotides away from $x$ on the same side, and [$x$-68, $x$+68] represents a bin from a site 68 nucleotides away from $x$ on the side of $x$ to a site 68 nucleotides away from $x$ on the other side of $x$.

In step S1223, determining the nucleosome center scores based on the nucleosome center scores corresponding to the base sites of the reference genome and the center score screening threshold includes the steps below.

(1) A maximum value of the nucleosome center scores is determined and a first position corresponding to the maximum value in the reference genome is determined.

(2) Nucleosome center scores of bases of particular data on two sides of the first position are zeroed, a maximum value is re-determined from the remaining nucleosome center scores after zeroing, and a position corresponding to the re-determined maximum value in the reference genome is determined, until the remaining nucleosome center scores are each less than a second target threshold, and screened positions are determined as candidate nucleosome center positions.

(3) The nucleosome center positions are determined based on the center score screening threshold and nucleosome center scores corresponding to the candidate nucleosome center positions.

The first position is a position of the reference genome corresponding to the maximum value of the nucleosome center scores. That is, the maximum value of the calculated nucleosome center scores is found, the position of the reference genome corresponding to the maximum value is recorded, and the nucleosome center scores of the bases of particular data on two sides (for example, 147 bases on each side) of the position are zeroed. This is because the found position is regarded as the nucleosome center position, and another nucleosome center position is impossible to exist within a range of at least 147 bases on each side of the nucleosome center position. Then, the position corresponding to the maximum value of the current nucleosome center scores is found again, and the step of zeroing the nucleosome center scores on two sides continues to be performed. The steps are cycled until the maximum value of the nucleosome center scores is less than the second target threshold. The positions screened based on the process are the nucleosome center positions. Theoretically, the count mean in the nucleosome linker region is higher than that in the nucleosome region. Based on the preceding formula, the nucleosome center scores should be greater than 2. To facilitate processing and improve accuracy, in an embodiment, the second target threshold may be set to 2.2. That is, when nucleosome center regions are screened, the steps are cycled until the maximum value of the nucleosome center scores is less than 2.2, and the iterative processing stops.

After all the candidate nucleosome center positions are obtained, the "nucleosome center scores" at some positions of the candidate nucleosome center positions are abnormally high since the corresponding second alignment counts are unreliable due to effects of various position factors such as alignment probability preference. Therefore, some of the candidate nucleosome center positions are used as the finally selected nucleosome center positions. The nucleosome center scores may be screened.

Table 2 below provides several score screening conditions and Pearson correlation coefficients between concentrations of fetal DNA and nucleosome ratios under the corresponding conditions.

Table 2

| Example | Score Screening Condition | Pearson Correlation Coefficient Between Concentrations of Fetal DNA and Nucleosome Ratios |
|---|---|---|
| 10 | The nucleosome region includes 147 bp, and the linker region includes 20 bp on a side and 20 bp on the other side. After the nucleosome center positions are screened, scores $\geq$ 2.2 are selected. | 0.2593 |

(continued)

| Example | Score Screening Condition | Pearson Correlation Coefficient Between Concentrations of Fetal DNA and Nucleosome Ratios |
|---|---|---|
| 11 | The nucleosome region includes 147 bp, and the linker region includes 20 bp on a side and 20 bp on the other side. After the nucleosome center positions are screened, scores in [2.2, 5] are selected. | 0.3543 |
| 12 | The nucleosome region includes 147 bp, and the linker region includes 20 bp on a side and 20 bp on the other side. After the nucleosome center positions are screened, scores in [2.2, 8] are selected. | 0.3340 |
| 13 | The nucleosome region includes 147 bp, and the linker region includes 20 bp on a side and 20 bp on the other side. After the nucleosome center positions are screened, scores in [10, ) are selected. | -0.0759 |
| 14 | The nucleosome region includes 147 bp, and the linker region includes 20 bp on a side and 20 bp on the other side. After the nucleosome center positions are screened, scores in [3, 6] are selected. | 0.3493 |
| 15 | The nucleosome region includes 137 bp, and the linker region includes 20 bp with a distance of 5 bp from the nucleosome region on a side and 20 bp with a distance of 5 bp from the nucleosome region on the other side. After the nucleosome center positions are screened, scores in [2.2, 5] are selected. | 0.3542 |
| 16 | The nucleosome region includes 137 bp, and the linker region includes 25 bp on a side and 25 bp on the other side. After the nucleosome center positions are screened, scores in [2.2, 5] are selected. | 0.3554 |

In Table 2, "score" refers to the "nucleosome center score", "bp" refers to the number of bases, "linker" refers to the nucleosome linker region, and "nucleosome ratio" refers to the "nucleosome ratio". The Pearson correlation coefficient between the "nucleosome ratios" and the reference concentrations of fetal DNA is used to primarily screen the nucleosome center positions and a method for calculating the nucleosome ratio. [10, ) represents a range of greater than or equal to 10, and the right parenthesis indicates an open interval.

As can be seen from Table 2, compared with no processing on the nucleosome region, the processing of truncating each of the left and right ends of the nucleosome region by 5 bases before the "nucleosome center scores" and the subsequent "nucleosome ratio" are calculated yields the higher Pearson correlation coefficient between the concentrations of fetal DNA and the nucleosome ratios.

In step S1223, based on the nucleosome center positions, the alignment and the summation are performed on the second alignment counts at the corresponding positions within all the nucleosome regions to obtain the summed second alignment counts.

In some embodiments, when alignment and superposition are performed according to the nucleosome regions, the alignment and the summation are performed within a range of 200 bases on the left and 200 bases on the right of the "nucleosome center position". That is, a vector of 401 elements is obtained, and the 201st element corresponds to the nucleosome center position. Such an operation has no effect on the calculation of the "nucleosome ratio". However, since a machine learning algorithm is subsequently introduced to build a model, information within a larger range around the nucleosome region is extracted to improve an amount of potential information and a prediction capability of the model.

After the nucleosome center positions are determined, for to-be-analyzed sample data, a count of reads whose starting positions are each position of the reference genome may be calculated by the preceding method. That is, the second alignment count is obtained. Then, based on the processing process of the nucleosome center positions, second alignment counts of 147 bases on the left and 147 bases on the right of the nucleosome center position are extracted. The operation is performed for all the nucleosome center positions. After the operation is completed, all extracted data are summed. That is, based on the nucleosome center positions, the alignment and the summation are performed on the second alignment counts at the corresponding positions within all the nucleosome regions. The corresponding positions within each nucleosome region include a particular number of bases on the left of the nucleosome center position and the particular number of bases on the right of the nucleosome center position. After repeated verification, 200 bases on the left and 200 bases on the right have the best processing effect. That is, a vector with a length of 295 bases is removed each time, where the 148th element of the vector is the second alignment count at the nucleosome center position, and the other elements of the vector are second alignment counts corresponding to positions on the left and right of the nucleosome center position. These vectors are summed. A summation result is shown in FIG. 6, which shows the distribution of a set of data subjected to the alignment and summation according to the nucleosome regions. As can be learned from FIG. 6, according to the aligned statistical data of the nucleosome regions obtained by the analysis and processing method, it can be seen with certainty that starting sites of the reads fall within the nucleosome center region at a far lower probability than within the nucleosome linker region, which is consistent with the biochemical principle and indirectly proves the reliability of the preceding analysis method.

A count mean of 147 bases in the nucleosome region (that is, 73 bases on the left and 73 bases on the right of the 148th element of the vector) corresponding to the summed vector is calculated, and the ratio of the count mean to a count mean in the nucleosome linker region on the left and right is calculated (for example, the nucleosome linker region includes the 74th to 93rd bases on the left and the 74th to 93rd bases on the right of the nucleosome center position). The ratio may be referred to as the "nucleosome ratio". That is, in the embodiments of the present application, for the alignment results determined based on nucleosome-related information, the higher the corresponding "nucleosome ratio", the higher the concentration of fetal DNA theoretically. A linear model is built according to a relationship between the nucleosome ratios and the reference concentrations of fetal DNA so that a relationship between the alignment results and the concentration of fetal DNA is obtained. Thus, the concentration of fetal DNA is determined based on the alignment results.

Specifically, a linear model between the second alignment counts and the concentration of fetal DNA may be built through machine learning. That the concentration of fetal DNA is determined based on the alignment results includes the steps below.

(1) Second training sample data are acquired. Each sample in the second training sample data is labeled with feature values and a target value, the feature values are normalized second alignment counts subjected to the dimensionality reduction, and the target value is an actual concentration of fetal DNA.

(2) Based on the second training sample data, a second concentration quantitation model of fetal DNA is built. The alignment results are input into the second concentration quantitation model of fetal DNA to obtain the concentration of fetal DNA.

A processing process of generating the second concentration quantitation model of fetal DNA is similar to a processing process of generating the first concentration quantitation model of fetal DNA, but the two models learn different feature values. Therefore, reference may be made to a process of creating the first concentration quantitation model of fetal DNA, and the details are not repeated here.

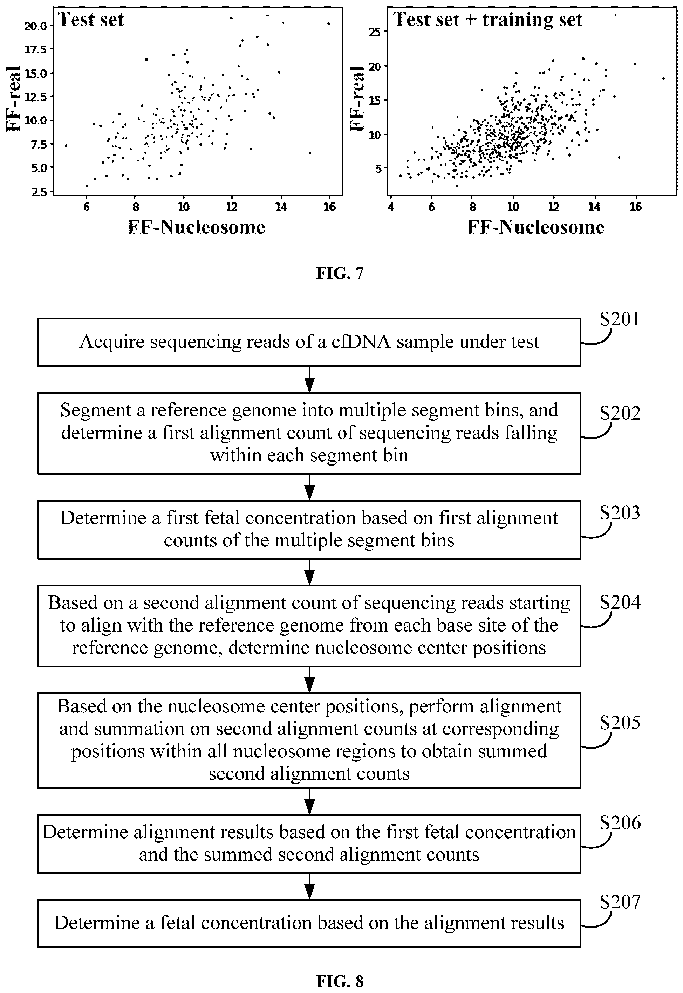

For example, the ratio of the count mean in the nucleosome region to the count mean in the nucleosome linker region is used as a parameter, and the linear model is built between the parameter and the known concentration of fetal DNA. Starting sites in single-molecule sequencing data have relatively low accuracy. Without removing bases with high error rates at two ends of the nucleosome region, the Pearson correlation coefficient is only about 0.3 (refer to Table 2). Therefore, in an embodiment, the counts at all the 401 positions in FIG. 6 are used as original features for machine learning modeling (a principal component analysis for dimensionality reduction + the elastic network for modeling) to estimate the concentration of fetal DNA. For example, for all of a certain volume of data such as 615 sets of data with the reference concentrations of fetal DNA, count vectors aligned and summed according to the nucleosome center positions are calculated. Each set corresponds to one vector of 401 elements, where the 401 elements are feature values as original input. The test set (30%) and the training set (70%) are randomly divided 100 times. The Pearson correlation coefficient between the estimated concentrations of fetal DNA and the actual concentrations of fetal DNA of the test set is 0.58, and $R^2$ is 0.33. FIG. 7 shows a model prediction effect after the test set and the training set are randomly divided at a time. In FIG. 7, the abscissa represents the concentrations of fetal DNA calculated by the nucleosome method, and the ordinate represents the reference concentrations of fetal DNA.

In some embodiments, original feature counts are processed so that the concentration of fetal DNA can be calculated based on the sequencing data obtained from various sequencing platforms, especially the single-molecule sequencing platform, with higher accuracy.

In an embodiment, original features are normalized. Specifically, 200 counts (second alignment counts) on the left and 200 counts (second alignment counts) on the right of the center position are each divided by a count of the center position to obtain 401 normalized features. Such normalization can reduce an effect of noise on the model and improve the performance of the model.

In another embodiment, new features are created based on the original features. For example, based on the preceding 401 original features, two new features are created. The first new feature is the ratio of a count mean of 20 leftmost positions to a count mean of 147 positions at the center, and the second new feature is a ratio of a count mean of 20 rightmost positions to the count mean of the 147 positions at the center. Thus, 403 features are formed.

In still another embodiment, principal component dimensionality reduction may be performed on the original features (where a principal component transformation model is obtained based on the training set and used for performing the same transformation on the test set) so that a complex relationship of associations of variables with each other is simplified, the model is simpler, and overfitting of the model is reduced.

The preceding three embodiments may be separately used to process the original features or may be combined to process the original features.

For example, after the new features are created based on the original features to form the 403 features, the principal component analysis for dimensionality reduction is performed on the 403 features (where the principal component transformation model is obtained based on the training set and used for performing the same transformation on the test set) so that the number of principal components and retained information are weighed and finally, the 403 features are dimensioned to 33 features with 90% of valid information retained.

During the machine learning modeling, to avoid the overfitting of the model, L1 and L2 regularization is performed on the model (where the L1 and L2 regularization is a regularization manner for adding additional terms to a loss function to prevent the overfitting of the model), and an optimal parameter combination for the L1 and L2 regularization is selected through iteration. During model training, to more accurately evaluate the model prediction effect, the test set (30%) and the training set (70%) are randomly divided 100 times to perform the model training. Finally, the Pearson correlation coefficient between the estimated concentrations of fetal DNA and the actual concentrations of fetal DNA of the test set is 0.58, and $R^2$ is 0.33. FIG. 7 shows a model prediction effect after the test set and the training set are randomly divided at a time.

Another embodiment of the present application provides another method for determining a concentration of fetal DNA. Referring to FIG. 8, the method may include the steps below.

In S201, multiple sequencing reads of a cfDNA sample under test are acquired.

The sequencing reads are as previously described. To save space, the details are not repeated here.

In S202, a reference genome is segmented into multiple segment bins, and a first alignment count of sequencing reads falling within each segment bin is determined.

In this step, to accurately obtain alignment results, the reference genome may be segmented according to a fixed length. For example, a segmentation length of the reference genome is determined according to features of the sequencing reads.

Some special chromosomes exist in the reference genome. These chromosomes may introduce a certain bias or other peculiarities into the process of determining the concentration of fetal DNA through alignment with the reference genome. In an implementation, segment bins of the special chromosomes (referred to as "particular chromosomes" below) are removed from the reference genome to reduce the bias caused by these chromosomes and minimize an effect of the peculiarities of these chromosomes on test results. In some embodiments, each particular chromosome includes at least one of chromosome X, chromosome Y, a mitochondrial chromosome, chromosome 13, chromosome 18, or chromosome 21. Segment bins corresponding to chromosome X and chromosome Y are removed so that differences of a male fetus and a female fetus in chromosome X and chromosome Y can be prevented from affecting results of the subsequent concentration quantitation model. Chromosome 13, chromosome 18, and chromosome 21 are removed due to relatively large probabilities of duplications and deletions of these chromosomes. One screening goal of NIPT is to screen the duplications and deletions of chromosome 13, chromosome 18, and chromosome 21. When chromosome 13, chromosome 18, and chromosome 21 are used as reference chromosomes, deviations may appear in data normalization statistics, for example, normalized alignment counts are affected, affecting calculation results and the accuracy of NIPT screening results.

In the embodiments of the present application, particular chromosomes may be adjusted according to expected requirements of detection of the concentration of fetal DNA. For example, chromosome X and chromosome Y are removed, chromosome 13, chromosome 18, and chromosome 21 are removed, or the mitochondrial chromosome is removed. Of course, multiple removals of the preceding removals may be performed simultaneously. For example, chromosome X, chromosome Y, the mitochondrial chromosome, chromosome 13, chromosome 18, and chromosome 21 or the corresponding segment bins are all removed so that the segment bins into which the reference genome is segmented do not include the segment bins corresponding to the preceding chromosomes.

In the present application, the particular chromosomes may be excluded from the segment bins in various manners. The segment bins obtained through segmentation may be screened based on the particular chromosomes. Alternatively, the particular chromosomes may be removed before segmentation. Specifically, the corresponding processing manner may be selected based on practical application requirements and is not limited in the present application.

In an embodiment, that the reference genome is segmented into the multiple segment bins includes: based on a preset segmentation length, segmenting the reference genome into multiple initial segment bins; and removing segment bins corresponding to the particular chromosome from the multiple initial segment bins to obtain the multiple segment bins. That is, after the reference genome is segmented into the multiple segment bins, the segment bins corresponding to the particular chromosome in the reference genome are removed and excluded from calculation. It is to be understood that the "segment bins corresponding to the particular chromosome" refer to multiple segment bins formed after the particular chromosome is segmented based on the preset segmentation length.

In another embodiment, that the reference genome is segmented into the multiple segment bins includes: removing the particular chromosome from the reference genome to obtain a removed reference genome; and based on the preset segmentation length, segmenting the removed reference genome into the multiple segment bins. That is, the particular chromosome is removed before the reference genome is segmented.

The preset segmentation length may be adjusted according to a data volume of data processing and an expected relative accuracy. For example, the reference genome may be segmented according to a length of every 50,000 bases. To make the obtained first alignment count more accurate, the first alignment count may be corrected to obtain a corrected first alignment count so that corrected first alignment counts are determined as the alignment results.

In some embodiments, correction includes GC correction. The GC correction is an operation in a bioinformatics analysis process. The GC correction is performed because gene sequencers and the corresponding sequencing processes have certain GC biases. For example, some sequencers have a higher probability of detecting high-GC reads, while some sequencers have a higher probability of detecting low-GC reads. In this embodiment, the concentration of fetal DNA is determined based on alignment probabilities in different regions of the reference genome. A GC bias of a sequencer affects the determination of actual alignment probabilities in different regions. Therefore, the GC correction is required to improve accuracy.

Correcting the first alignment count to obtain the corrected first alignment count includes: based on the first alignment count of each segment bin and a mean of first alignment counts of the multiple segment bins, normalizing the first alignment count of each segment bin to obtain a normalized first alignment count of each segment bin; and performing the GC correction on the normalized first alignment count of each segment bin to obtain a GC-corrected first alignment count. The mean of the first alignment counts of the multiple segment bins refers to the ratio of a sum of the first alignment counts of the segment bins to the total number of the segment bins.

After the first alignment counts of the multiple segment bins are obtained, to facilitate calculation and processing, the first alignment counts of the multiple segment bins are normalized by the corresponding formula:
the normalized first alignment count of each segment bin = the first alignment count of each segment bin/the mean of the first alignment counts of the multiple segment bins.

After the first alignment count of each segment bin is obtained, the GC correction is performed to obtain the GC-corrected first alignment count.

In an embodiment, performing the GC correction on the normalized first alignment count of each segment bin to obtain the GC-corrected first alignment count includes the steps below.

(1) A first relationship curve is generated based on a GC content and the normalized first alignment count of each segment bin.

In this embodiment, the GC content may be represented by various parameters, such as the GC content or a GC content median of each segment bin, or may be represented by other parameters that can reflect the GC content of the segment bin.

In the embodiments of the present application, GC filtering may be performed on the segment bins forming the first relationship curve. For example, the segment bins are filtered according to GC contents so that some abnormal or statistically insignificant segment bins are filtered out, thereby optimizing the first relationship curve and reducing data processing resources occupied during data processing.

In an embodiment, the GC filtering is implemented in the manner below. Before the first relationship curve is generated based on the GC content and the normalized first alignment count of each segment bin, the following processing is performed: each segment bin is filtered based on the GC content, and each segment bin whose GC content does not satisfy a preset requirement is removed so that the first relationship curve is generated based on the GC content of each segment bin after GC filtering and the normalized first alignment count of each segment bin.

In another embodiment, the GC filtering is implemented in the manner below. In the process of, based on the first relationship curve, performing the GC correction on the normalized first alignment count, each segment bin is filtered based on the GC content, and based on the first relationship curve, the GC correction is performed on the normalized first alignment count of each segment bin after GC filtering to obtain the GC-corrected first alignment count.

In the preceding embodiments, as an example, the first relationship curve is generated based on the GC content and the normalized first alignment count of each segment bin in the manner below. An x-axis represents the GC content of each segment bin (that is, a proportion of GC bases corresponding to the segment bin of the reference genome), and a y-axis represents the normalized first alignment count of each segment bin. The segment bins and data on GC contents of the segment bins are integrated. That is, a scatter plot is generated according to the GC content and the normalized first alignment count of each segment bin. Scatter data in the scatter plot are smoothed to obtain a smooth curve. The scatter data in the scatter plot may be smoothed using an algorithm such as LOWESS to obtain the smooth curve, which is the first relationship curve, for example, $y_s = f(x_s)$.

Specifically, as an example, the first relationship curve may be generated in the manner below. The abscissa is divided into different sections, a GC content median of points in the same section is calculated, the GC content median represents a value corresponding to the section, and all GC content medians are plotted and smoothed to obtain the first relationship curve. In another example, all data may be directly smoothed. That is, all data points are considered and smoothed to obtain the smooth curve. Thus, a Spearman correlation coefficient obtained through a test set is higher. It is to be noted that this smoothing method is also applicable to the subsequent alignment probability smoothing and correction.

As a possible implementation, before the first relationship curve is generated based on the GC content and the normalized first alignment count of each segment bin, the step below is further included.

Each segment bin is filtered based on the GC content of each segment bin so that the first relationship curve is generated based on the GC content of each segment bin after GC filtering and the normalized first alignment count of each segment bin.

As another possible implementation, before the first relationship curve is generated based on the GC content and the normalized first alignment count of each segment bin, the step below is further included.

Based on the first relationship curve, performing the GC correction on the normalized first alignment count to obtain the GC-corrected first alignment count includes the steps below.

Each segment bin is filtered based on the GC content of each segment bin, and based on the first relationship curve, the GC correction is performed on the normalized first alignment count of each segment bin after GC filtering to obtain the GC-corrected first alignment count.

(2) Based on the first relationship curve, the GC correction is performed on the normalized first alignment count to obtain the GC-corrected first alignment count.

In some embodiments, the GC correction is performed according to a subtraction or division relation between the normalized first alignment count of each segment bin and the GC content of each segment bin to determine the GC-corrected first alignment count.

In an example, the GC correction method is "subtraction", and the corresponding correction formula is $y_{GC} = y - f(x)$. In another example, the GC correction method is "division", and the corresponding correction formula is $y_{GC} = y / f(x)$.

In the above two correction formulas, x represents the GC content of the segment bin, y represents the normalized first alignment count of the segment bin, and $y_{GC}$ represents the GC-corrected normalized first alignment count.

In some embodiments, the correction further includes alignment probability correction to improve the accuracy of the obtained first alignment count. That is, in some embodiments, the correction includes both the GC correction and the alignment probability correction.

In an implementation of the embodiment of the present application, correcting the first alignment count to obtain the corrected first alignment count further includes: cutting the reference genome based on a particular sliding window length to obtain sequence cuts, aligning the sequence cuts with the reference genome, counting a first alignment count of sequence cuts falling within each segment bin, and determining the first alignment count as a sliding window alignment count of each segment bin; based on the sliding window alignment count, determining a normalized sliding window alignment count of each segment bin; and performing the alignment probability correction based on the normalized sliding window alignment count and the normalized first alignment count of each segment bin to determine a first alignment count subjected to the alignment probability correction.

In the embodiments of the present application, the first alignment count is corrected by the "alignment probability correction" method based on the following consideration: if a different region of a reference sequence of the reference genome is cut according to a particular length as a sequencing read and then aligned with the reference genome, the sequencing read is aligned to different segment bins for different times. Since sequences in some regions are similar to those at multiple positions of reference sequences of the reference genome, these regions are more easily aligned. For example, reference sequences of a human genome hg 19 are cut according to a step size of 2 bases and a sliding window of 37 bases to obtain sequence cuts, and these sequence cuts are aligned with the human reference genome hg19. An alignment count of sequence cuts in a different segment bin of the human reference genome is counted, which may be referred to as the "sliding window alignment count". Specifically, the particular sliding window length may be considered based on a practical application scenario. For example, the particular sliding window length is determined based on the characteristics of the reference genome or the characteristics of the sample under test. After the particular sliding window length is determined, the reference genome is cut, the sequence cuts are aligned with the reference genome, the first alignment count of sequence cuts falling within each segment bin is counted, and the first alignment count is determined as the sliding window alignment count. After the sliding window alignment count is obtained, to facilitate calculation and processing, the normalized sliding window alignment count of each segment bin is determined based on the sliding window alignment count; and the alignment probability correction is performed on the normalized sliding window alignment count. For the alignment probability correction method, refer to the preceding GC correction method.

In an embodiment, performing the alignment probability correction based on the normalized sliding window alignment count and the normalized first alignment count of each segment bin to determine the first alignment count subjected to the alignment probability correction includes the steps below.

(1) A second relationship curve is generated based on the normalized sliding window alignment count of each segment bin and the normalized first alignment count corresponding to each segment bin.

The normalized sliding window alignment count of each segment bin may be represented by various parameters, such as the normalized sliding window alignment count or a median of sliding window alignment counts of each segment bin, or may be represented by other parameters that can reflect the normalized sliding window alignment count of each segment bin.

In the embodiments of the present application, normalized sliding window alignment counts forming the second relationship curve may be filtered so that some abnormal or statistically insignificant segment bins are filtered out, thereby optimizing the second relationship curve and improving the accuracy of related data processing based on the second relationship curve.

In an embodiment, the normalized sliding window alignment counts forming the second relationship curve are filtered in the manner below. Before the second relationship curve is generated based on the normalized sliding window alignment count of each segment bin and the normalized first alignment count corresponding to each segment bin, the following processing is performed: the segment bins are filtered based on the normalized sliding window alignment counts, and segment bins whose normalized sliding window alignment counts are each not less than a first target threshold are obtained so that the second relationship curve is generated for the segment bins whose normalized sliding window alignment counts are each not less than the first target threshold.

In an embodiment, the normalized sliding window alignment counts forming the second relationship curve are filtered in the manner below. In the process of, based on the second relationship curve, performing the alignment probability correction on the normalized first alignment count to obtain the first alignment count subjected to the alignment probability correction, the segment bins are filtered based on the normalized sliding window alignment counts, and second relationship curve sections of the second relationship curve where normalized sliding window alignment counts are each not less than the first target threshold are retained to obtain a second relationship curve after alignment probability filtering; and based on the second relationship curve after alignment probability filtering, the alignment probability correction is performed on the normalized sliding window alignment count of each segment bin to obtain the first alignment count subjected to the alignment probability correction.

In the preceding embodiments, based on repeated verification, the first target threshold may be 0.8. That is, each segment bin whose normalized sliding window alignment count is less than 0.8 is removed.

In the preceding embodiments, as an example, the second relationship curve is generated based on the normalized sliding window alignment count of each segment bin and the normalized first alignment count corresponding to each segment bin in the manner below. An x-axis represents the normalized sliding window alignment count of each segment bin, and a y-axis represents the normalized first alignment count corresponding to each segment bin. The segment bins and data on the GC contents of the segment bins are integrated. That is, a scatter plot is generated according to the GC content of each segment bin and the normalized first alignment count corresponding to each segment bin. Scatter data in the scatter plot are smoothed to obtain a smooth curve. For example, the scatter data in the scatter plot may be smoothed using the LOWESS algorithm.

(2) Based on the second relationship curve, the alignment probability correction is performed on the normalized first alignment count to obtain the first alignment count subjected to the alignment probability correction.

In some embodiments, the alignment probability correction is performed according to a subtraction or division relation between the normalized first alignment count of each segment bin and the sliding window alignment count of each segment bin to determine the first alignment count subjected to the alignment probability correction.

In an example, the alignment probability correction method is "subtraction", and the corresponding correction formula is $y_{alignment\ probability} = y - f(x)$. In another example, the alignment probability correction method is "division", and the corresponding correction formula is $y_{alignment\ probability} = y/f(x)$.

In the above two correction formulas, x represents the normalized sliding window alignment count of each segment bin, y represents the normalized first alignment count of each segment bin, and $y_{alignment\ probability}$ represents the normalized first alignment count subjected to the alignment probability correction.

In S203, a first concentration of fetal DNA is determined based on the first alignment counts of the multiple segment bins.

In the embodiments of the present application, the concentration of fetal DNA may be determined by a machine learning method in conjunction with a neural network. In an implementation of the embodiment of the present application, that the concentration of fetal DNA is determined based on the alignment results includes the steps below.

First training sample data are obtained, where each sample in the first training sample data is labeled with first feature values and a first target value, the first feature values are sample alignment counts, and the first target value is an actual concentration of fetal DNA.

Based on a particular model structure, machine learning modeling is performed on the first training sample data to obtain a first concentration quantitation model of fetal DNA.

The alignment results are input into the first concentration quantitation model of fetal DNA to obtain the first concentration of fetal DNA and the first concentration of fetal DNA is determined as the concentration of fetal DNA.

In some embodiments, based on the particular model structure, performing the machine learning modeling on the first training sample data to obtain the first concentration quantitation model of fetal DNA includes the steps below.

The first training sample data are divided into a training set and a test set. The machine learning modeling is performed based on the training set to obtain an initial model.

The test set is processed based on the initial model to obtain a predicted concentration of fetal DNA in each test sample in the test set.

The predicted concentration of fetal DNA is compared with an actual concentration of fetal DNA in each test sample in the test set to obtain a comparison result.

A model parameter of the initial model is adjusted based on the comparison result to obtain the first concentration quantitation model of fetal DNA.

The first training sample data may be data in a database of cfDNA in peripheral blood of pregnant women and with known concentrations of fetal DNA, where the data are obtained based on various sequencing platforms including a single-molecule sequencing platform. For example, a certain number of samples with known concentrations of fetal DNA are used, the reference sequences of chromosomes of the reference genome are segmented into bins (for example, bins of 5,000 bases, 50,000 bases, 100,000 bases, 300,000 bases, or 800,000 bases, for example), and alignment counts of alignment data of each sample in different segment bins are separately counted. The alignment counts are normalized and subjected to the GC correction and the alignment probability correction by the LOWESS method, the corrected normalized alignment counts of the different segment bins are used as the feature values, and the known actual concentration of fetal DNA in each sample is used as the target value, and a particular model is used for learning and modeling. The particular model may be a classification model, a convolutional neural network model, or the like. The particular model structure is not limited in the embodiments of the present application as long as the training sample data can be learned and the concentration of fetal DNA can be predicted. A model learning and training process is an iterative process. Therefore, training samples may be divided into the training set and the test set. The accuracy of the currently trained model is tested by the test set. If the accuracy is lower than a set accuracy threshold, the model parameter in the model structure of the current model is adjusted by using the test set until an error between an output concentration of fetal DNA of the obtained model and the actual concentration of fetal DNA satisfies a corresponding error range.

In the case of a relatively small number of samples for machine learning and relatively many feature values (for example, about three thousand to six hundred thousand features, depending on a size of the segment bin of the reference sequences of the chromosomes), overfitting cannot be avoided in a dimensionality reduction or learning process. In the finally built concentration quantitation model of fetal DNA, a prediction effect of the concentration of fetal DNA in the test set has a certain deviation from the actual concentration of fetal DNA. For example, in the concentration quantitation model of fetal DNA built using 615 samples with known concentrations of fetal DNA, $R^2$ corresponding to the test set of the model is only about 0.3, and a Pearson correlation coefficient between actual values and predicted values is about 0.5. However, when the alignment counts are normalized and corrected (the GC correction or a combination of the GC correction and the alignment probability correction) by the preceding method according to the embodiment of the present application, the correlation coefficient between the predicted values and the actual values of the test set increases. As can be known from the comparison between the modeling directly performed by using the normalized alignment counts of the segment bins and the modeling performed by using the normalized alignment counts of the segment bins subjected to the GC correction and the alignment probability correction, the modeling after normalization, the GC correction, and the alignment probability correction has a better effect, that is, the correlation coefficient between the predicted values and the actual values of the test set is higher.

In another embodiment, that the concentration of fetal DNA is determined based on the alignment results includes the steps below.

The alignment results are input into a first preset model to obtain an initial concentration of fetal DNA.

The initial concentration of fetal DNA is corrected based on a second preset model to obtain the concentration of fetal DNA.

The first preset model is a linear relationship model built based on alignment results in second sample data and an initial concentration of fetal DNA corresponding to the alignment results, and the second sample data include the alignment results of alignment of sequencing reads of a cfDNA sample with the reference genome and the concentration of fetal DNA corresponding to the alignment results. The second preset model is a model obtained after the first preset model is processed based on constants determined through linear fitting.

For example, the first preset model may be $FF1 = (\overrightarrow{data})$, where $\overrightarrow{data}$ represents the alignment results, and FF1 represents the initial concentration of fetal DNA. The second preset model may be $FF2 = FF1 \times c + d$, where c and d represent the constants determined through linear fitting, and FF2 represents the concentration of fetal DNA.

For example, SeqFF is used for model building and estimation of the concentration of fetal DNA according to differences in alignment probabilities of samples in different bins of the reference sequences of the reference genome. The SeqFF method uses two machine learning models, one is WRSC and the other is Enet. The final output concentration of fetal DNA is a mean of predicted values of the two models. When the SeqFF model is tested using data from the sequencing platforms, especially the single-molecule sequencing platform, the WRSC model has a better effect than Enet. That is, a linear correlation coefficient between concentrations of fetal DNA estimated by WRSC and actual concentrations of fetal DNA is higher than that of Enet or SeqFF. Therefore, a model parameter involved in the WRSC algorithm is preferentially considered. Specifically, reference may be made to FIGS. 2 and 3. FIG. 2 is a schematic diagram of a relationship between concentrations of fetal DNA calculated by an Enet model and reference concentrations of fetal DNA according to an embodiment of the present application. FIG. 3 is a schematic diagram of a relationship between concentrations of fetal DNA calculated by a WRSC model and reference concentrations of fetal DNA according to an embodiment of the present application. As can be seen from the figures, a positive correlation between the concentrations of fetal DNA calculated by the WRSC model and the reference concentrations of fetal DNA is superior to a positive correlation between the concentrations of fetal DNA calculated by the Enet model and the reference concentrations of fetal DNA.

For example, in the WRSC model, the reference sequences of the chromosome are segmented according to a bin of a particular number of bases, such as 50,000 bases. After training using the WRSC algorithm, a linear relationship between coverage densities in different bins of the reference sequences and the concentration of fetal DNA is found, as shown by the following formula. In the following formula, FF represents the concentration of fetal DNA, $\overrightarrow{data}$ represents the coverage densities in different bins of the reference sequences, $\vec{A}$ represents a constant vector whose dimensions are equal to those of $\overrightarrow{data}$, B represents a parameter matrix for linear transformation, and c and d represent constants:

$$FF = \text{sum}\{(\overrightarrow{data} - \vec{A}) \times B\} \times c + d$$

SeqFF is a model trained based on data of second-generation sequencing whose sequencing principle has a relatively large difference from that of single-molecule sequencing. Therefore, if the preceding model parameters of SeqFF are directly used to calculate the concentration of fetal DNA based on data obtained by other sequencing platforms including the single-molecule sequencing platform, the calculated concentration of fetal DNA has a relatively large difference from the actual concentration of fetal DNA. 615 NIPT clinical samples with the known concentrations of fetal DNA in the preceding embodiment are still used as examples for analysis and testing. Data in original alignment files (such as SAM files) from the other sequencing platforms including the single-molecule sequencing platform are directly processed by using the algorithm and parameters of SeqFF. The Pearson correlation coefficient between the obtained intermediate parameters and the actual concentrations of fetal DNA is 0.714.

A linear relationship between calculated values of the concentrations of fetal DNA calculated by the SeqFF model and the actual concentrations of fetal DNA is relatively good. Therefore, the model parameters included in sum $\{(\overrightarrow{data} - \vec{A}) \times B\}$ in the above formula may be used to calculate an intermediate parameter of the concentration of fetal DNA. Then, values of c and d are self-fitted in a linear regression manner so that model parameters applicable to the current various sequencing platforms including the single-molecule sequencing platform are obtained.

615 NIPT clinical samples with the known concentrations of fetal DNA in the preceding embodiment are still used as examples for analysis and testing. Instead, the first alignment count of sequencing reads falling within each segment bin is used for analysis; regions of chromosomes X, Y, M, 13, 18, and 21 and the mitochondrial chromosome of the reference genome where alignment probabilities are abnormal are removed for analysis; the correction method is optimized (the GC correction or a combination of the GC correction and the alignment probability correction). The data are re-analyzed by using the optimized parameters. The Pearson correlation coefficient between the intermediate parameters and the concentrations of fetal DNA is 0.755. The test set (30%) and the training set (70%) are randomly divided 100 times to establish a linear model and determine the above constant parameters c and d. An average prediction $R^2$ value corresponding to the test set and obtained from 100 times of modeling is 0.561.

It is to be noted that when the linear model is truly built, the test set and the training set are divided to evaluate, by limited data, the reliability of the model currently built based on the training set randomly divided. Therefore, the test set (30%) and the training set (70%) are randomly divided 100 times. The average prediction $R^2$ value corresponding to the test set and obtained from 100 times of modeling is 0.561.

Nine examples in which the concentration of fetal DNA is determined according to differences in probabilities of alignment of maternal and fetal cfDNA to different bins of the reference genome are shown above in Table 1.

As can be seen from Table 1, compared with the algorithm (whose Pearson correlation coefficient is 0.71425) in Example 1, the other algorithms have Pearson correlation coefficients all greater than 0.74, and the algorithms in Example 6, Example 8, and Example 9 have Pearson correlation coefficients greater than 0.75. Compared with the other algorithms, the algorithm in Example 8 can obtain the highest Pearson correlation coefficient of 0.75471.

After correction, a relationship between the calculated concentrations of fetal DNA and the reference concentrations of fetal DNA in Example 8 is shown in FIG. 5.

In S204, based on a second alignment count of times each base site of the reference genome acts as starting positions of alignment of sequencing reads with the reference genome, nucleosome center positions are determined.

This step includes the steps below.

The second alignment count of times each base site of the reference genome acts as the starting positions of alignment of the sequencing reads with the reference genome is determined, and based on the second alignment count, a nucleosome center score corresponding to each base site of the reference genome is calculated.

The nucleosome center positions are determined based on nucleosome center scores corresponding to base sites of the reference genome and a center score screening threshold.

In some embodiments, calculating the nucleosome center score corresponding to each base site of the reference genome includes the steps below.

(1) A first count mean of second alignment counts of a first particular number of bases on the left and the first particular number of bases on the right of each base site of the reference genome is calculated.

(2) A second count mean of second alignment counts of a second particular number of bases on the left and the second particular number of bases on the right of each base site of the reference genome is calculated.

(3) The nucleosome center score corresponding to each base site is determined according to the first count mean and the second count mean.

The first particular number and the second particular number are determined based on the number of bases in a nucleosome region and the number of bases in a nucleosome linker region. With $x$ as one base site, the first particular number = (the number of bases in the nucleosome region - 1)/2, and the second particular number = (the number of bases in the nucleosome region - 1)/2 + the number of bases in the nucleosome linker region/2.

For example, as reported in the literature, the length of the nucleosome region is 147 bases, and the linker region includes about 20 bases at one end and about 20 bases at the other end of the nucleosome. The first particular number = (147 - 1)/2 = 73, and the second particular number = (147 - 1)/2 + 20 = 93. That is, assuming that $x$ represents a nucleosome center position, 73 bases on the left of the nucleosome center position and 73 bases on the right of the nucleosome center position form the nucleosome region, and the 74th base to the 93rd base on the left of the nucleosome center position and the 74th base to the 93rd base on the right of the nucleosome center position form the nucleosome linker region.

By the above formula, the ratio of a sum of a count mean in a bin of the 74th to 93rd bases on the left of a to-be-observed position and a count mean in a bin of the 74th to 93rd bases on the right of the to-be-observed position to a count mean in a bin of 73 bases on the left and 73 bases on the right of the to-be-observed position is calculated. Apparently, in the proximity of a particular nucleosome region, the closer the to-be-observed position to the nucleosome center position, the larger the nucleosome center score.

Further, the nucleosome center score corresponding to each site is determined according to the first count mean and the second count mean. The calculation formula of the nucleosome center score may be expressed as follows:

$$\text{the nucleosome center score} =$$

$$\frac{\text{count mean in a bin } [x-93, x-74] + \text{count mean in a bin } [x+93, x+74]}{\text{count mean in a bin } [x-73, x+73]} .$$

In the formula, $x$ represents the base site, $[x$-93, $x$-74] represents a bin from a site 93 nucleotides away from $x$ on a side of $x$ to a site 74 nucleotides away from $x$ on the same side, $[x$+93, $x$+74] represents a bin from a site 93 nucleotides away from $x$ on the other side of $x$ to a site 74 nucleotides away from $x$ on the same side, and $[x$-73, $x$+73] represents a bin from a site 73 nucleotides away from $x$ on the side of $x$ to a site 73 nucleotides away from $x$ on the other side of x.

In some embodiments, considering that a sequencing error rate of a single-molecule sequencer tends to be higher than an error rate of the second-generation sequencing, when the probability of each base in the nucleosome region being the nucleosome center position is analyzed and compared, regions with relatively high error rates at two ends of a sequence are cut off so that the accuracy and efficiency of determination of the nucleosome center position can be improved.

In some embodiments, the "nucleosome center score" and the subsequent "nucleosome ratio" are calculated after the left and right ends of the nucleosome region are each truncated by n bases, where n is a natural number less than or equal to 5. For example, n is 1, 2, 3, 4, or 5.

In this case, the calculation formula of the nucleosome center score may be expressed as follows:

$$\text{the nucleosome center score} =$$

$$\frac{count\ mean\ in\ a\ bin[x-93,x-74+n]+count\ mean\ in\ a\ bin\ [x+93,x+74-n]}{count\ mean\ in\ a\ bin[x-73+n,x+73-n]}.$$

In the formula, $x$ represents the base site, [$x$-93, $x$-74+$n$] represents a bin from a site 93 nucleotides away from $x$ on a side of $x$ to a site (74-$n$) nucleotides away from $x$ on the same side, [$x$+93, $x$+74-$n$] represents a bin from a site 93 nucleotides away from $x$ on the other side of $x$ to a site (74-$n$) nucleotides away from $x$ on the same side, and [$x$-73+$n$, $x$+73-$n$] represents a bin from a site (73-$n$) nucleotides away from $x$ on the side of $x$ to a site (73-$n$) nucleotides away from $x$ on the other side of $x$.

For example, when n = 5, the calculation formula of the nucleosome center score may be expressed as follows:

$$\text{the nucleosome center score} =$$

$$\frac{count\ mean\ in\ a\ bin\ [x-93, x-69] + count\ mean\ in\ a\ bin\ [x+93, x+69]}{count\ mean\ in\ a\ bin\ [x-68, x+68]}$$

In the formula, $x$ represents the base site, [$x$-93, $x$-69] represents a bin from a site 93 nucleotides away from $x$ on a side of $x$ to a site 69 nucleotides away from $x$ on the same side, [$x$+93, $x$+69] represents a bin from a site 93 nucleotides away from $x$ on the other side of $x$ to a site 69 nucleotides away from $x$ on the same side, and [$x$-68, $x$+68] represents a bin from a site 68 nucleotides away from $x$ on the side of $x$ to a site 68 nucleotides away from $x$ on the other side of $x$.

Determining the nucleosome center scores based on the nucleosome center scores corresponding to the base sites of the reference genome and the center score screening threshold includes the steps below.

(1) A maximum value of the nucleosome center scores is determined and a first position corresponding to the maximum value in the reference genome is determined.

(2) Nucleosome center scores of bases of particular data on two sides of the first position are zeroed, a maximum value is re-determined from the remaining nucleosome center scores after zeroing, and a position corresponding to the re-determined maximum value in the reference genome is determined, until the remaining nucleosome center scores are each less than a second target threshold, and screened positions are determined as candidate nucleosome center positions.

(3) The nucleosome center positions are determined based on the center score screening threshold and nucleosome center scores corresponding to the candidate nucleosome center positions.

The first position is a position of the reference genome corresponding to the maximum value of the nucleosome center scores. That is, the maximum value of the calculated nucleosome center scores is found, the position of the reference genome corresponding to the maximum value is recorded, and the nucleosome center scores of the bases of particular data on two sides (for example, 147 bases on each side) of the position are zeroed. This is because the found position is regarded as the nucleosome center position, and another nucleosome center position is impossible to exist within a range of at least 147 bases on each side of the nucleosome center position. Then, the position corresponding to the maximum value of the current nucleosome center scores is found again, and the step of zeroing the nucleosome center scores on two sides continues to be performed. The steps are cycled until the maximum value of the nucleosome center scores is less than the second target threshold. The positions screened based on the process are the nucleosome center positions. Theoretically, the count mean in the nucleosome linker region is higher than that in the nucleosome region. Based on the preceding formula, the nucleosome center scores should be greater than 2. To facilitate processing and improve accuracy, in an embodiment, the second target threshold may be set to 2.2. That is, when nucleosome center regions are screened, the steps are cycled until the maximum value of the nucleosome center scores is less than 2.2, and the iterative processing stops.

After all the candidate nucleosome center positions are obtained, the "nucleosome center scores" at some positions of the candidate nucleosome center positions are abnormally high since the corresponding second alignment counts are unreliable due to effects of various position factors such as alignment probability preference. Therefore, some of the candidate nucleosome center positions are used as the finally selected nucleosome center positions. The nucleosome center scores may be screened.

Table 2 above provides several score screening conditions and Pearson correlation coefficients between concentrations of fetal DNA and nucleosome ratios under the corresponding conditions.

In S205, based on the nucleosome center positions, alignment and summation are performed on second alignment counts at corresponding positions within all nucleosome regions to obtain summed second alignment counts.

In some embodiments, when alignment and superposition are performed according to the nucleosome regions, the alignment and the summation are performed within a range of 200 bases on the left and 200 bases on the right of the "nucleosome center position". That is, a vector of 401 elements is obtained, and the 201st element corresponds to the nucleosome center position. Such an operation has no effect on the calculation of the "nucleosome ratio". However, since a machine learning algorithm is subsequently introduced to build a model, information within a larger range around the nucleosome region is extracted to improve an amount of potential information and a prediction capability of the model.

After the nucleosome center positions are determined, for to-be-analyzed sample data, a count of reads whose starting positions are each position of the reference genome may be calculated by the preceding method. That is, the second alignment count is obtained. Then, based on the processing process of the nucleosome center positions, second alignment counts of 147 bases on the left and 147 bases on the right of the nucleosome center position are extracted. The operation is performed for all the nucleosome center positions.

In S206, the alignment results are determined based on the first concentration of fetal DNA and the summed second alignment counts.

In this embodiment, feature values provided in the second processing mode are processed by the same method as when the second processing mode is used alone. The difference is that the concentration of fetal DNA predicted in the first processing mode is added as one feature and subjected to a principal component analysis for dimensionality reduction together with the feature values. During model training, to ensure the stability of the model and the reliability of a model evaluation result, the test set (30%) and the training set (70%) are randomly divided 100 times. An elastic net model is also subjected to L1 and L2 regularization, and an optimal parameter combination for the L1 and L2 regularization is selected through iteration. The specific effects of different models are shown in Tables 3 and 4. In the following tables, r2_test_enst represents $R^2$ of the test set when the enst model is used for modeling; r2_train_enst represents $R^2$ of the training set when the enst model is used for modeling; corr_test_enst represents a Pearson correlation coefficient of the test set when the enst model is used for modeling; and corr_train_enst represents a Pearson correlation coefficient of the training set when the enst model is used for modeling. The same is true for other models.

It is to be noted that "the test set (30%) and the training set (70%) are randomly divided 100 times", and means and standard deviations (STDs) of $R^2$ and Pearson correlation coefficients obtained through 100 times of division are calculated to evaluate, by limited data, the reliability of the model currently built based on the training set randomly divided and the model effect. During modeling, when the training set and the test set are divided only once and the modeling is performed, $R^2$ is 0.61 and the Pearson correlation coefficient is 0.79. As can be seen from the tables below, when the training set and the test set are randomly divided 100 times, the means of the obtained $R^2$ and Pearson correlation coefficients are 0.61 and 0.78 respectively, with STD values being 0.04 and 0.03, respectively. Therefore, the model effect ($R^2$ of 0.61 and a Pearson correlation coefficient of 0.79) obtained when the modeling is performed with a single division is reliable.

Table 3

|  | Mean |
| --- | --- |
| r2_test_enst | 0.6033 |
| r2_train_enst | 0.6478 |
| corr_test_enst | 0.7835 |
| corr_train_enst | 0.8063 |
| r2_test_lasso | 0.6078 |
| r2_train_lasso | 0.6565 |
| corr_test_lasso | 0.7839 |
| corr train _lasso | 0.8111 |
| r2_test_liner | 0.6059 |
| r2_train_liner | 0.6614 |

(continued)

|  | Mean |
|---|---|
| corr_test_liner | 0.7824 |
| corr train _liner | 0.8132 |
| r2_test_linerstep | 0.6028 |
| r2_train_linerstep | 0.6286 |
| corr_test_linerstep | 0.7810 |
|  |  |
| corr_train_linerstep | 0.7927 |
| r2_test_ridge | 0.6066 |
| r2_train_ridge | 0.6604 |
| corr_test _ridge | 0.7824 |
| corr_train_ridge | 0.8132 |
| r2_test_keras | 0.5565 |
| r2_train_keras | 0.6408 |
| corr_test_keras | 0.7724 |
| corr_train_keras | 0.8209 |
| r2_test_lgb | 0.5340 |
| r2_train_lgb | 0.9127 |
| corr_test_lgb | 0.7488 |
| corr_train_lgb | 0.9724 |

Table 4

|  | STD |
|---|---|
| r2_test_enst | 0.0390 |
| r2_train_enst | 0.0184 |
| corr_test_enst | 0.0277 |
| corr_train_enst | 0.0112 |
| r2_test_lasso | 0.0399 |
| r2_train_lasso | 0.0212 |
| corr_test_lasso | 0.0271 |
| corr_train_lasso | 0.0117 |
| r2_test_liner | 0.0403 |
| r2_train_liner | 0.0179 |
|  |  |
| corr_test_liner | 0.0272 |
| corr_train_liner | 0.0110 |
| r2_test_linerstep | 0.0427 |
| r2_train_linerstep | 0.0201 |
| corr_test_linerstep | 0.0291 |
| corr_train_linerstep | 0.0126 |

(continued)

| r2_test_ridge | 0.0400 |
|---|---|
| r2_train_ridge | 0.0170 |
| corr_test_ridge | 0.0272 |
| corr_train_ridge | 0.0110 |
| r2_test_keras | 0.0628 |
| r2_train_keras | 0.0497 |
| corr_test_keras | 0.0264 |
| corr_train_keras | 0.0182 |
| r2_test_lgb | 0.0464 |
| r2_train_lgb | 0.0276 |
| corr_test_lgb | 0.0353 |
| corr_train_lgb | 0.0075 |

As a possible implementation of the present application, in some embodiments, determining the alignment results based on the first concentration of fetal DNA and the summed second alignment counts includes the steps below. The dimensionality reduction is performed on the summed second alignment counts corresponding to base sites within the nucleosome regions to obtain normalized second alignment counts subjected to the dimensionality reduction; and the alignment results are determined based on the first concentration of fetal DNA and the normalized second alignment counts subjected to the dimensionality reduction. Alternatively, initial alignment results are determined based on the first concentration of fetal DNA and the summed second alignment counts; the dimensionality reduction is performed on the initial alignment results, and initial alignment results subjected to the dimensionality reduction are determined as the alignment results.

For example, 401 original features (that is, 401 counts obtained through the alignment and summation based on the nucleosome center positions) in the second processing mode are combined with the concentration of fetal DNA directly obtained by the WRSC method in the first determination mode into 402 parameter data. The parameter data, as originally input feature values, are subjected to the principal component analysis for dimensionality reduction, and the modeling is performed using a machine learning algorithm. Machine learning models may be various models such as the elastic net, a least absolute shrinkage and selection operator (Lasso), linear regression, stepwise regression, ridge regression, Keras, and lightGBM. Compared with the other models, the ridge regression has the best effect, and $R^2$ of the test set for estimation of the concentration of fetal DNA is 0.61.

In S207, the concentration of fetal DNA is determined based on the alignment results.

In another embodiment, that the concentration of fetal DNA is determined based on the alignment results includes the steps below.

Second training sample data are acquired, where each sample in the second training sample data is labeled with feature values and a target value, the feature values are normalized second alignment counts subjected to the dimensionality reduction, and the target value is an actual concentration of fetal DNA. Based on the second training sample data, a second concentration quantitation model of fetal DNA is built. The alignment results are input into the second concentration quantitation model of fetal DNA to obtain the concentration of fetal DNA.

This embodiment is implemented based on the determination of the concentration of fetal DNA according to the differences in the probabilities of alignment of the maternal and fetal cfDNA to different bins of sequences of the reference genome (simply referred to as the first determination mode for ease of description) and the determination of the concentration of fetal DNA according to differences in the distribution of two ends of the maternal and fetal cfDNA at nucleosome positions (simply referred to as a second determination mode) in the preceding embodiments. Since the concentration of fetal DNA is determined according to different principles in the two modes, parameters in the two modes are combined to determine the concentration of fetal DNA so that the feature values include more valid information and the capability of estimating the concentration of fetal DNA is higher. For example, the 401 original features (that is, the 401 counts obtained through the alignment and summation based on the nucleosome center positions) in the second processing mode are combined with the concentration of fetal DNA directly obtained by the WRSC method in the first determination mode into the 402 parameter data. The parameter data, as the originally input feature values, are subjected to the principal component analysis for dimensionality reduction, and the modeling is performed using the machine learning algorithm, facilitating the obtaining of a more accurate concentration of fetal DNA.

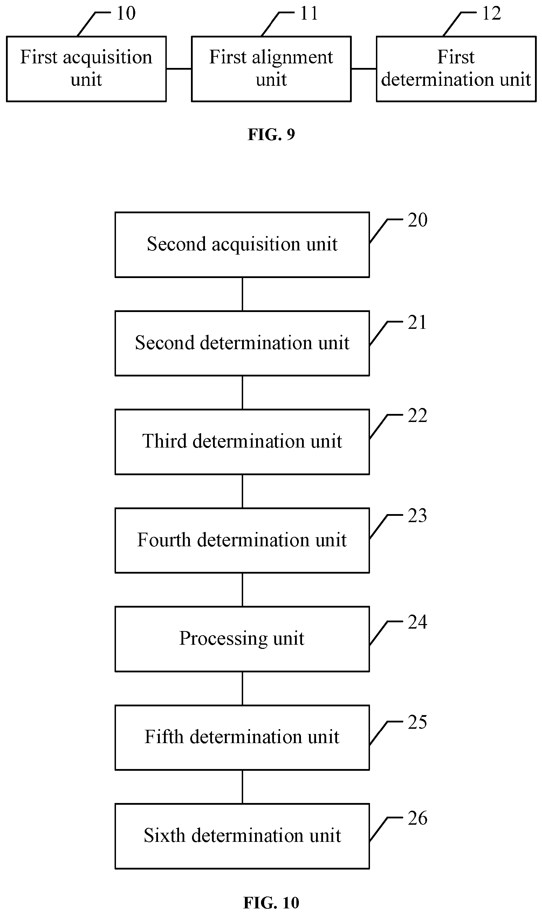

Based on the preceding embodiments, another embodiment of the present application provides an apparatus for determining a concentration of fetal DNA. Referring to FIG. 9, the apparatus may include a first acquisition unit 10, a first alignment unit 11, and a first determination unit 12.

The first acquisition unit 10 is configured to acquire multiple sequencing reads of a cfDNA sample under test.

The first alignment unit 11 is configured to align the multiple sequencing reads with a reference genome to obtain alignment results.

The first determination unit 12 is configured to determine the concentration of fetal DNA based on the alignment results.

As a possible implementation of the present application, the first alignment unit includes a first segmentation subunit and a first determination subunit.

The first segmentation subunit is configured to segment the reference genome into multiple segment bins.

The first determination subunit is configured to determine a first alignment count of sequencing reads falling within each segment bin, and determine first alignment counts of the multiple segment bins as the alignment results.

As a possible implementation of the present application, the first segmentation subunit is configured to perform the operations below.

Based on a preset segmentation length, the reference genome is segmented into multiple initial segment bins.

Segment bins corresponding to a particular chromosome are removed from the multiple initial segment bins to obtain the multiple segment bins.

Alternatively, a particular chromosome is removed from the reference genome to obtain a removed reference genome.

Based on a preset segmentation length, the removed reference genome is segmented into the multiple segment bins.

The particular chromosome includes at least one of chromosome X, chromosome Y, a mitochondrial chromosome, chromosome 13, chromosome 18, or chromosome 21.

As a possible implementation of the present application, the apparatus further includes a first correction unit.

The first correction unit is configured to correct the first alignment count to obtain a corrected first alignment count, where corrected first alignment counts of the multiple segment bins are determined as the alignment results.

As a possible implementation of the present application, the first correction unit includes a first processing subunit and a GC correction subunit.

The first processing subunit is configured to, based on the first alignment count of each segment bin and a mean of the first alignment counts of the multiple segment bins, normalize the first alignment count of each segment bin to obtain a normalized first alignment count of each segment bin.

The GC correction subunit is configured to perform GC correction on the normalized first alignment count of each segment bin to obtain a GC-corrected first alignment count.

As a possible implementation of the present application, the GC correction subunit is configured to perform the operations below.

A first relationship curve is generated based on a GC content and the normalized first alignment count of each segment bin.

Based on the first relationship curve, the GC correction is performed on the normalized first alignment count to obtain the GC-corrected first alignment count.

Before the first relationship curve is generated based on the GC content and the normalized first alignment count of each segment bin, the operation below is further included.

Each segment bin is filtered based on the GC content of each segment bin so that the first relationship curve is generated based on the GC content of each segment bin after GC filtering and the normalized first alignment count of each segment bin.

Alternatively, based on the first relationship curve, the GC correction is performed on the normalized first alignment count in the manner below to obtain the GC-corrected first alignment count.

Each segment bin is filtered based on the GC content of each segment bin, and based on the first relationship curve, the GC correction is performed on the normalized first alignment count of each segment bin after GC filtering to obtain the GC-corrected first alignment count.

Specifically, the GC correction involves determining the GC-corrected first alignment count according to a subtraction or division relation between the normalized first alignment count of each segment bin and the GC content of each segment bin.

In another embodiment, the first correction unit further includes a cutting subunit, a second determination subunit, and a third determination subunit.

The cutting subunit is configured to cut the reference genome based on a particular sliding window length to obtain sequence cuts, align the sequence cuts with the reference genome, count a first alignment count of sequence cuts falling within each segment bin, and determine the first alignment count as a sliding window alignment count of each segment bin.

The second determination subunit is configured to, based on the sliding window alignment count, determine a normalized sliding window alignment count of each segment bin.

The third determination subunit is configured to perform alignment probability correction based on the normalized sliding window alignment count and the normalized first alignment count of each segment bin to determine a first alignment count subjected to the alignment probability correction.

As a possible implementation of the present application, the third determination subunit is configured to perform the operations below.

A second relationship curve is generated based on the normalized sliding window alignment count of each segment bin and the normalized first alignment count corresponding to each segment bin.

Based on the second relationship curve, the alignment probability correction is performed on the normalized first alignment count to obtain the first alignment count subjected to the alignment probability correction.

In an embodiment, before the second relationship curve is generated based on the normalized sliding window alignment count of each segment bin and the normalized first alignment count corresponding to each segment bin, the operation below is further included.

The segment bins are filtered based on normalized sliding window alignment counts of the segment bins and segment bins whose normalized sliding window alignment counts are each not less than a first target threshold are obtained so that the second relationship curve is generated for the segment bins whose normalized sliding window alignment counts are each not less than the first target threshold.

Alternatively, based on the second relationship curve, the alignment probability correction is performed on the normalized first alignment count in the manner below to obtain the first alignment count subjected to the alignment probability correction.

The segment bins are filtered based on normalized sliding window alignment counts of the segment bins and second relationship curve sections of the second relationship curve where normalized sliding window alignment counts are each not less than a first target threshold are retained to obtain a second relationship curve after alignment probability filtering.

Based on the second relationship curve after alignment probability filtering, the alignment probability correction is performed on the normalized sliding window alignment count to obtain the first alignment count subjected to the alignment probability correction.

As a possible implementation of the present application, the first determination unit includes a first sample acquisition subunit, a first modeling subunit, and a model processing subunit.

The first sample acquisition subunit is configured to obtain first training sample data, where each sample in the first training sample data is labeled with first feature values and a first target value, the first feature values are alignment counts, and the first target value is an actual concentration of fetal DNA.

The first modeling subunit is configured to, based on a particular model structure, perform machine learning modeling on the first training sample data to obtain a first concentration quantitation model of fetal DNA.

The model processing subunit is configured to input the alignment results into the first concentration quantitation model of fetal DNA to obtain a first concentration of fetal DNA and determine the first concentration of fetal DNA as the concentration of fetal DNA.

As a possible implementation of the present application, the first modeling subunit is configured to perform the operations below.

The first training sample data are divided into a training set and a test set.

The machine learning modeling is performed based on the training set to obtain an initial model.

The test set is processed based on the initial model to obtain a predicted concentration of fetal DNA in each test sample in the test set.

The predicted concentration of fetal DNA is compared with an actual concentration of fetal DNA in each test sample in the test set to obtain a comparison result.

A model parameter of the initial model is adjusted based on the comparison result to obtain the first concentration quantitation model of fetal DNA.

Specifically, the first determination unit is further configured to perform the operations below.

The alignment results are input into a first preset model to obtain an initial concentration of fetal DNA, where the first preset model is a linear relationship model built based on alignment results in second sample data and an initial concentration of fetal DNA corresponding to the alignment results, and the second sample data include the alignment results of alignment of sequencing reads of a cfDNA sample with the reference genome and the concentration of fetal DNA corresponding to the alignment results.

The initial concentration of fetal DNA is corrected based on a second preset model to obtain the concentration of fetal DNA, where the second preset model is a model obtained after the first preset model is processed based on constants determined through linear fitting.

In another embodiment, the first alignment unit is configured to perform the operations below.

A second alignment count of times each base site of the reference genome acts as starting positions of alignment of sequencing reads with the reference genome is determined, and based on the second alignment count, a nucleosome center score corresponding to each base site of the reference genome is calculated.

Nucleosome center positions are determined based on nucleosome center scores corresponding to base sites of the reference genome and a center score screening threshold.

Based on the nucleosome center positions, alignment and summation are performed on second alignment counts at corresponding positions within all nucleosome regions to obtain summed second alignment counts.

Dimensionality reduction is performed on the summed second alignment counts to obtain normalized second alignment counts subjected to the dimensionality reduction, and the normalized second alignment counts subjected to the dimensionality reduction are determined as the alignment results.

The nucleosome center score corresponding to each base site of the reference genome is calculated in the manner below.

A first count mean of second alignment counts of a first particular number of bases on the left and the first particular number of bases on the right of each base site of the reference genome is calculated.

A second count mean of second alignment counts of a second particular number of bases on the left and the second particular number of bases on the right of each base site of the reference genome is calculated.

The nucleosome center score corresponding to each base site is determined according to the first count mean and the second count mean.

As a possible implementation of the present application, the nucleosome center score is calculated by the following formula:

$$\text{the nucleosome center score} =$$

$$\frac{count\ mean\ in\ a\ bin[x-93,x-74+n]+count\ mean\ in\ a\ bin\ [x+93,x+74-n]}{count\ mean\ in\ a\ bin[x-73+n,x+73-n]}.$$

In the formula, $x$ represents the base site, $[x-93, x-74+n]$ represents a bin from a site 93 nucleotides away from $x$ on a side of $x$ to a site $(74-n)$ nucleotides away from $x$ on the same side, $[x+93, x+74-n]$ represents a bin from a site 93 nucleotides away from $x$ on the other side of $x$ to a site $(74-n)$ nucleotides away from $x$ on the same side, $[x-73+n, x+73-n]$ represents a bin from a site $(73-n)$ nucleotides away from $x$ on the side of $x$ to a site $(73-n)$ nucleotides away from $x$ on the other side of $x$, and $n$ represents a natural number less than or equal to 5.

As a possible implementation of the present application, the nucleosome center scores are determined based on the nucleosome center scores corresponding to the base sites of the reference genome and the center score screening threshold in the manner below.

A maximum value of the nucleosome center scores is determined and a first position corresponding to the maximum value in the reference genome is determined.

Nucleosome center scores of bases of particular data on two sides of the first position are zeroed, a maximum value is re-determined from the remaining nucleosome center scores after zeroing, and a position corresponding to the re-determined maximum value in the reference genome is determined, until the remaining nucleosome center scores are each less than a second target threshold, and screened positions are determined as candidate nucleosome center positions.

The nucleosome center positions are determined based on the center score screening threshold and nucleosome center scores corresponding to the candidate nucleosome center positions.

Specifically, in the step of, based on the nucleosome center positions, performing the alignment and the summation on the second alignment counts at the corresponding positions within all the nucleosome regions, the corresponding positions within each nucleosome region include a particular number of bases on the left and the particular number of bases on the right of each of the nucleosome center positions.

In an embodiment, the first determination unit is further configured to perform the operations below.

Second training sample data are acquired. Each sample in the second training sample data is labeled with feature values and a target value, the feature values are normalized second alignment counts subjected to the dimensionality reduction, and the target value is an actual concentration of fetal DNA.

Based on the second training sample data, a second concentration quantitation model of fetal DNA is built.

The alignment results are input into the second concentration quantitation model of fetal DNA to obtain the concentration of fetal DNA.

Based on the preceding embodiments, another embodiment of the present application provides another apparatus for determining a concentration of fetal DNA. Referring to FIG. 10, the apparatus may include a second acquisition unit 20, a second determination unit 21, a third determination unit 22, a fourth determination unit 23, a processing unit 24, a fifth determination unit 25, and a sixth determination unit 26.

The second acquisition unit 20 is configured to acquire multiple sequencing reads of a cfDNA sample under test.

The second determination unit 21 is configured to segment a reference genome into multiple segment bins and determine a first alignment count of sequencing reads falling within each segment bin.

The third determination unit 22 is configured to determine a first concentration of fetal DNA based on first alignment counts of the multiple segment bins.

The fourth determination unit 23 is configured to, based on a second alignment count of times each base site of the reference genome acts as starting positions of alignment of sequencing reads with the reference genome, determine nucleosome center positions.

The processing unit 24 is configured to, based on the nucleosome center positions, perform alignment and summation on second alignment counts at corresponding positions within all nucleosome regions to obtain summed second alignment counts.

The fifth determination unit 25 is configured to determine alignment results based on the first concentration of fetal DNA and the summed second alignment counts.

The sixth determination unit 26 is configured to determine the concentration of fetal DNA based on the alignment results.

In some embodiments, the second determination unit includes a second segmentation subunit.

The second segmentation subunit is configured to perform the operations below.

Based on a preset segmentation length, the reference genome is segmented into multiple initial segment bins.

Segment bins corresponding to a particular chromosome are removed from the multiple initial segment bins to obtain the multiple segment bins.

Alternatively, a particular chromosome is removed from the reference genome to obtain a removed reference genome.

Based on a preset segmentation length, the removed reference genome is segmented into the multiple segment bins.

In some embodiments, the apparatus further includes a second correction unit.

The second correction unit is configured to correct the first alignment count to obtain a corrected first alignment count, where the first concentration of fetal DNA is determined based on corrected first alignment counts of the multiple segment bins.

In some embodiments, the second correction unit includes a second processing subunit and a GC correction subunit.

The second processing subunit is configured to, based on the first alignment count of each segment bin and a mean of the first alignment counts of the multiple segment bins, normalize the first alignment count of each segment bin to obtain a normalized first alignment count of each segment bin.

The GC correction subunit is configured to perform GC correction on the normalized first alignment count of each segment bin to obtain a GC-corrected first alignment count.

As a possible implementation of the present application, the GC correction subunit is configured to perform the operations below.

Based on a GC content and the normalized first alignment count of each segment bin, a first relationship curve matching a particular coordinate system is generated.

Based on the first relationship curve, the GC correction is performed on the normalized first alignment count to obtain the GC-corrected first alignment count.

In some embodiments, the second correction subunit is further configured to perform the operations below.

The reference genome is cut based on a particular sliding window length to obtain sequence cuts, the sequence cuts are aligned with the reference genome, a first alignment count of sequence cuts falling within each segment bin is counted, and the first alignment count is determined as a sliding window alignment count of each segment bin.

Based on the sliding window alignment count, a normalized sliding window alignment count of each segment bin is determined.

Alignment probability correction is performed based on the normalized sliding window alignment count and the normalized first alignment count of each segment bin to determine a first alignment count subjected to the alignment probability correction.

In some embodiments, the third determination unit is configured to perform the operations below.

First training sample data are obtained, where each sample in the first training sample data is labeled with first feature values and a first target value, the first feature values are alignment counts, and the first target value is an actual concentration of fetal DNA.

Based on a particular model structure, machine learning modeling is performed on the first training sample data to obtain a first concentration quantitation model of fetal DNA.

The first alignment counts are input into the first concentration quantitation model of fetal DNA to obtain the first concentration of fetal DNA.

As a possible implementation of the present application, the third determination unit is further configured to perform the operations below.

The first alignment counts are input into a first preset model to obtain an initial concentration of fetal DNA, where the first preset model is a linear relationship model built based on alignment results in second sample data and an initial concentration of fetal DNA corresponding to the alignment results, and the second sample data include the alignment results of alignment of sequencing reads of a cfDNA sample with the reference genome and the concentration of fetal DNA corresponding to the alignment results.

The initial concentration of fetal DNA is corrected based on a second preset model to obtain the first concentration of fetal DNA, where the second preset model is a model obtained after the first preset model is processed based on constants determined through linear fitting.

In some embodiments, the fourth determination unit includes a score calculation subunit and a center position determination subunit.

The score calculation subunit is configured to determine the second alignment count of times each base site of the reference genome acts as the starting positions of alignment of the sequencing reads with the reference genome, and based on the second alignment count, calculate a nucleosome center score corresponding to each base site of the reference genome.

The center position determination subunit is configured to determine the nucleosome center positions based on nucleosome center scores corresponding to base sites of the reference genome and a center score screening threshold.

In some embodiments, the fifth determination unit is configured to perform the operations below.

Dimensionality reduction is performed on the summed second alignment counts corresponding to base sites within the nucleosome regions to obtain normalized second alignment counts subjected to the dimensionality reduction.

The alignment results are determined based on the first concentration of fetal DNA and the normalized second alignment counts subjected to the dimensionality reduction.

Alternatively, initial alignment results are determined based on the first concentration of fetal DNA and the summed second alignment counts.

Dimensionality reduction is performed on the initial alignment results, and initial alignment results subjected to the dimensionality reduction are determined as the alignment results.

It is to be noted that for execution processes of the units of the apparatus for determining the concentration of fetal DNA of this embodiment, reference may be made to the method for determining the concentration of fetal DNA in FIG. 8. The details are not repeated here.

Another embodiment of the present application provides a readable storage medium. The readable storage medium stores a computer program which, when executed by a processor, causes the processor to perform the steps of the method for determining the concentration of fetal DNA according to any one of the preceding embodiments.

Another embodiment of the present application provides an electronic device. The electronic device may include a memory and a processor.

The memory is configured to store application programs and data generated by the running of the application programs.

The processor is configured to execute the application programs to perform the method for determining the concentration of fetal DNA according to any one of the preceding embodiments.

It is to be noted that for specific implementations of the processor in this embodiment, reference may be made to the corresponding content described above. The details are not repeated here.

Embodiments in this Description are described in a progressive manner. Each embodiment focuses on differences from other embodiments. The same or similar parts in the embodiments can be referred to by each other. The apparatus disclosed in the embodiments corresponds to the method disclosed in the embodiments and thus is described relatively simply. For the related part, reference may be made to the description of the method.

Those skilled in the art should be further aware that the units and algorithm steps in the examples described in conjunction with the embodiments disclosed herein can be implemented by electronic hardware, computer software, or a combination thereof. To clearly illustrate the interchangeability of hardware and software, the composition and steps of the examples have been described generally in terms of functionality in the preceding description. Whether these functions are performed by hardware or software depends on the particular application and design constraints of the technical solutions. Those skilled in the art can use different methods to implement the described functions for each particular application, but such implementation should not be considered as exceeding the scope of the present application.

The steps of the method or algorithm described in conjunction with the embodiments disclosed herein may be directly implemented by hardware, software modules executed by a processor, or a combination thereof. The software modules may be stored in a random-access memory (RAM), a memory, a read-only memory (ROM), an electrically programmable ROM, an electrically erasable programmable ROM, a register, a hard disk, a removable disk, a compact disc ROM (CD-ROM), or any other form of storage medium known in the art.

The preceding description of the disclosed embodiments enables those skilled in the art to implement or use the present application. Various modifications to these embodiments are apparent to those skilled in the art. The general principles defined herein can be implemented in other embodiments without departing from the spirit or scope of the present application. Therefore, the present application is not limited to the embodiments disclosed herein but is to accord with the widest scope consistent with the principles and novel features disclosed herein.

**Claims**

1. A method for determining a concentration of fetal DNA, comprising:

   acquiring a plurality of sequencing reads of a cell-free DNA (cfDNA) sample under test;
   aligning the plurality of sequencing reads with a reference genome to obtain alignment results; and
   determining the concentration of fetal DNA based on the alignment results.

2. The method according to claim 1, wherein aligning the plurality of sequencing reads with the reference genome to obtain the alignment results comprises:

   segmenting the reference genome into a plurality of segment bins; and
   determining, among the plurality of sequencing reads, a first alignment count of sequencing reads falling within each segment bin of the plurality of segment bins, and determining first alignment counts of the plurality of segment bins as the alignment results.

3. The method according to claim 2, wherein segmenting the reference genome into the plurality of segment bins comprises one of:

   based on a preset segmentation length, segmenting the reference genome into a plurality of initial segment bins; and
   removing segment bins corresponding to a particular chromosome from the plurality of initial segment bins to obtain the plurality of segment bins;
   or
   removing a particular chromosome from the reference genome to obtain a removed reference genome; and
   based on a preset segmentation length, segmenting the removed reference genome into the plurality of segment bins.

4. The method according to claim 3, wherein the particular chromosome comprises at least one of chromosome X, chromosome Y, a mitochondrial chromosome, chromosome 13, chromosome 18, or chromosome 21.

5. The method according to any one of claims 2 to 4, further comprising:
   correcting the first alignment count to obtain a corrected first alignment count, and determining corrected first alignment counts of the plurality of segment bins as the alignment results.

6. The method according to claim 5, wherein correcting the first alignment count to obtain the corrected first alignment count comprises:

   based on the first alignment count of each segment bin and a mean of the first alignment counts of the plurality of segment bins, normalizing the first alignment count of each segment bin to obtain a normalized first alignment count of each segment bin; and
   performing guanine-cytosine (GC) correction on the normalized first alignment count of each segment bin to obtain a GC-corrected first alignment count.

7. The method according to claim 6, wherein performing the GC correction on the normalized first alignment count of each segment bin to obtain the GC-corrected first alignment count comprises:

   generating a first relationship curve based on a GC content and the normalized first alignment count of each segment bin; and
   based on the first relationship curve, performing the GC correction on the normalized first alignment count to obtain the GC-corrected first alignment count.

8. The method according to claim 7, wherein
before generating the first relationship curve based on the GC content and the normalized first alignment count of each segment bin, the method further comprises:

filtering each segment bin based on the GC content of each segment bin to enable the first relationship curve to be generated based on the GC content of each segment bin after GC filtering and the normalized first alignment count of each segment bin;
or
based on the first relationship curve, performing the GC correction on the normalized first alignment count to obtain the GC-corrected first alignment count comprises:
filtering each segment bin based on the GC content of each segment bin, and based on the first relationship curve, performing the GC correction on the normalized first alignment count of each segment bin after GC filtering to obtain the GC-corrected first alignment count.

9. The method according to claim 7 or 8, wherein the GC correction involves determining the GC-corrected first alignment count according to a subtraction or division relation between the normalized first alignment count of each segment bin and the GC content of each segment bin.

10. The method according to any one of claims 5 to 9, wherein correcting the first alignment count to obtain the corrected first alignment count further comprises:

cutting the reference genome based on a particular sliding window length to obtain sequence cuts, aligning the sequence cuts with the reference genome, counting a first alignment count of sequence cuts falling within each segment bin, and determining the first alignment count as a sliding window alignment count of each segment bin;
based on the sliding window alignment count, determining a normalized sliding window alignment count of each segment bin; and
performing alignment probability correction based on the normalized sliding window alignment count and the normalized first alignment count of each segment bin to determine a first alignment count subjected to the alignment probability correction.

11. The method according to claim 10, wherein performing the alignment probability correction based on the normalized sliding window alignment count and the normalized first alignment count of each segment bin to determine the first alignment count subjected to the alignment probability correction comprises:

generating a second relationship curve based on the normalized sliding window alignment count of each segment bin and the normalized first alignment count corresponding to each segment bin; and
based on the second relationship curve, performing the alignment probability correction on the normalized first alignment count to obtain the first alignment count subjected to the alignment probability correction.

12. The method according to claim 11, wherein
before generating the second relationship curve based on the normalized sliding window alignment count of each segment bin and the normalized first alignment count corresponding to each segment bin, the method further comprises:

filtering the plurality of segment bins based on normalized sliding window alignment counts of the plurality of segment bins and obtaining segment bins whose normalized sliding window alignment counts are each not less than a first target threshold to enable the second relationship curve to be generated for the segment bins whose normalized sliding window alignment counts are each not less than the first target threshold;
or
based on the second relationship curve, performing the alignment probability correction on the normalized first alignment count to obtain the first alignment count subjected to the alignment probability correction comprises:

filtering the plurality of segment bins based on normalized sliding window alignment counts of the plurality of segment bins and retaining second relationship curve sections of the second relationship curve where normalized sliding window alignment counts are each not less than a first target threshold to obtain a second relationship curve after alignment probability filtering; and
based on the second relationship curve after alignment probability filtering, performing the alignment probability correction on the normalized sliding window alignment count to obtain the first alignment count subjected to the alignment probability correction.

13. The method according to any one of claims 2 to 12, wherein determining the concentration of fetal DNA based on the alignment results comprises:

obtaining first training sample data, wherein each sample in the first training sample data is labeled with first feature values and a first target value, the first feature values are sample alignment counts, and the first target value is an actual concentration of fetal DNA;

based on a particular model structure, performing machine learning modeling on the first training sample data to obtain a first concentration quantitation model of fetal DNA; and

inputting the alignment results into the first concentration quantitation model of fetal DNA to obtain a first concentration of fetal DNA and determining the first concentration of fetal DNA as the concentration of fetal DNA.

14. The method according to claim 13, wherein based on the particular model structure, performing the machine learning modeling on the first training sample data to obtain the first concentration quantitation model of fetal DNA comprises:

dividing the first training sample data into a training set and a test set;

performing the machine learning modeling based on the training set to obtain an initial model;

processing the test set based on the initial model to obtain a predicted concentration of fetal DNA in each test sample in the test set;

comparing the predicted concentration of fetal DNA with an actual concentration of fetal DNA in each test sample in the test set to obtain a comparison result; and

adjusting a model parameter of the initial model based on the comparison result to obtain the first concentration quantitation model of fetal DNA.

15. The method according to any one of claims 2 to 12, wherein determining the concentration of fetal DNA based on the alignment results comprises:

inputting the alignment results into a first preset model to obtain an initial concentration of fetal DNA, wherein the first preset model is a linear relationship model built based on alignment results in second sample data and an initial concentration of fetal DNA corresponding to the alignment results, and the second sample data comprise the alignment results of alignment of sequencing reads of a cfDNA sample with the reference genome and the concentration of fetal DNA corresponding to the alignment results; and

correcting the initial concentration of fetal DNA based on a second preset model to obtain the concentration of fetal DNA, wherein the second preset model is a model obtained after the first preset model is processed based on constants determined through linear fitting.

16. The method according to claim 1, wherein aligning the sequencing reads with the reference genome to obtain the alignment results comprises:

determining a second alignment count of times each base site of the reference genome acts as starting positions of alignment of sequencing reads with the reference genome, and based on the second alignment count, calculating a nucleosome center score corresponding to each base site of the reference genome;

determining nucleosome center positions based on nucleosome center scores corresponding to base sites of the reference genome and a center score screening threshold;

based on the nucleosome center positions, performing alignment and summation on second alignment counts at corresponding positions within all nucleosome regions to obtain summed second alignment counts; and

performing dimensionality reduction on the summed second alignment counts to obtain normalized second alignment counts subjected to the dimensionality reduction, and determining the normalized second alignment counts subjected to the dimensionality reduction as the alignment results.

17. The method according to claim 16, wherein calculating the nucleosome center score corresponding to each base site of the reference genome comprises:

calculating a first count mean of second alignment counts of a first particular number of bases on the left and the first particular number of bases on the right of each base site of the reference genome;

calculating a second count mean of second alignment counts of a second particular number of bases on the left and the second particular number of bases on the right of each base site of the reference genome; and

determining the nucleosome center score corresponding to each base site according to the first count mean and the second count mean.

18. The method according to claim 17, wherein the nucleosome center score is calculated by the following formula:

$$\text{the nucleosome center score} = \frac{count\ mean\ in\ a\ bin[x-93, x-74+n] + count\ mean\ in\ a\ bin\ [x+93, x+74-n]}{count\ mean\ in\ a\ bin[x-73+n, x+73-n]},$$

wherein x represents the base site, [x-93, x-74+n] represents a bin from a site 93 nucleotides away from x on a side of x to a site (74-n) nucleotides away from x on the same side, [x+93, x+74-n] represents a bin from a site 93 nucleotides away from x on another side of x to a site (74-n) nucleotides away from x on the same side, [x-73+n, x+73-n] represents a bin from a site (73-n) nucleotides away from x on the side of x to a site (73-n) nucleotides away from x on the another side of x, and n represents a natural number less than or equal to 5.

19. The method according to any one of claims 16 to 18, wherein determining the nucleosome center scores based on the nucleosome center scores corresponding to the base sites of the reference genome and the center score screening threshold comprises:

determining a maximum value of the nucleosome center scores and determining a first position corresponding to the maximum value in the reference genome;
zeroing nucleosome center scores of bases of particular data on two sides of the first position, redetermining a maximum value from remaining nucleosome center scores after zeroing, and determining a position corresponding to the re-determined maximum value in the reference genome, until the remaining nucleosome center scores are each less than a second target threshold, and determining screened positions as candidate nucleosome center positions; and
determining the nucleosome center positions based on the center score screening threshold and nucleosome center scores corresponding to the candidate nucleosome center positions.

20. The method according to any one of claims 16 to 18, wherein in a step of, based on the nucleosome center positions, performing the alignment and the summation on the second alignment counts at the corresponding positions within all the nucleosome regions, the corresponding positions within each nucleosome region comprise:
a particular number of bases on the left and the particular number of bases on the right of each of the nucleosome center positions.

21. The method according to any one of claims 16 to 20, wherein determining the concentration of fetal DNA based on the alignment results comprises:

acquiring second training sample data, wherein each sample in the second training sample data is labeled with feature values and a target value, the feature values are normalized second alignment counts subjected to the dimensionality reduction, and the target value is an actual concentration of fetal DNA;
based on the second training sample data, building a second concentration quantitation model of fetal DNA; and
inputting the alignment results into the second concentration quantitation model of fetal DNA to obtain the concentration of fetal DNA.

22. A method for determining a concentration of fetal DNA, comprising:

acquiring a plurality of sequencing reads of a cell-free DNA (cfDNA) sample under test;
segmenting a reference genome into a plurality of segment bins, and determining, among the plurality of sequencing reads, a first alignment count of sequencing reads falling within each segment bin of the plurality of segment bins;
determining a first concentration of fetal DNA based on first alignment counts of the plurality of segment bins;
based on a second alignment count of times each base site of the reference genome acts as starting positions of alignment of sequencing reads with the reference genome, determining nucleosome center positions;
based on the nucleosome center positions, performing alignment and summation on second alignment counts at corresponding positions within all nucleosome regions to obtain summed second alignment counts;
determining alignment results based on the first concentration of fetal DNA and the summed second alignment counts; and
determining the concentration of fetal DNA based on the alignment results.

23. The method according to claim 22, wherein segmenting the reference genome into the plurality of segment bins comprises one of:

based on a preset segmentation length, segmenting the reference genome into a plurality of initial segment bins; and
removing segment bins corresponding to a particular chromosome from the plurality of initial segment bins to obtain the plurality of segment bins;
or
removing a particular chromosome from the reference genome to obtain a removed reference genome; and
based on a preset segmentation length, segmenting the removed reference genome into the plurality of segment bins.

24. The method according to claim 23, wherein the particular chromosome comprises at least one of chromosome X, chromosome Y, a mitochondrial chromosome, chromosome 13, chromosome 18, or chromosome 21.

25. The method according to any one of claims 22 to 24, wherein before determining the first concentration of fetal DNA based on the first alignment counts of the plurality of segment bins, the method further comprises:
correcting the first alignment count to obtain a corrected first alignment count, and determining the first concentration of fetal DNA based on corrected first alignment counts of the plurality of segment bins.

26. The method according to claim 25, wherein correcting the first alignment count to obtain the corrected first alignment count comprises:

based on the first alignment count of each segment bin and a mean of the first alignment counts of the plurality of segment bins, normalizing the first alignment count of each segment bin to obtain a normalized first alignment count of each segment bin; and
performing guanine-cytosine (GC) correction on the normalized first alignment count of each segment bin to obtain a GC-corrected first alignment count.

27. The method according to claim 26, wherein performing the GC correction on the normalized first alignment count of each segment bin to obtain the GC-corrected first alignment count comprises:

generating a first relationship curve based on a GC content and the normalized first alignment count of each segment bin; and
based on the first relationship curve, performing the GC correction on the normalized first alignment count to obtain the GC-corrected first alignment count.

28. The method according to claim 27, wherein
before generating the first relationship curve based on the GC content and the normalized first alignment count of each segment bin, the method further comprises:

filtering each segment bin based on the GC content of each segment bin to enable the first relationship curve to be generated based on the GC content of each segment bin after GC filtering and the normalized first alignment count of each segment bin;
or
based on the first relationship curve, performing the GC correction on the normalized first alignment count to obtain the GC-corrected first alignment count comprises:
filtering each segment bin based on the GC content of each segment bin, and based on the first relationship curve, performing the GC correction on the normalized first alignment count of each segment bin after GC filtering to obtain the GC-corrected first alignment count.

29. The method according to claim 27 or 28, wherein the GC correction involves determining the GC-corrected first alignment count according to a subtraction or division relation between the normalized first alignment count of each segment bin and the GC content of each segment bin.

30. The method according to any one of claims 25 to 29, wherein correcting the first alignment count to obtain the corrected first alignment count further comprises:

cutting the reference genome based on a particular sliding window length to obtain sequence cuts, aligning the sequence cuts with the reference genome, counting a first alignment count of sequence cuts falling within each segment bin, and determining the first alignment count as a sliding window alignment count of each segment bin; based on the sliding window alignment count, determining a normalized sliding window alignment count of each segment bin; and performing alignment probability correction based on the normalized sliding window alignment count and the normalized first alignment count of each segment bin to determine a first alignment count subjected to the alignment probability correction.

31. The method according to claim 30, wherein performing the alignment probability correction based on the normalized sliding window alignment count and the normalized first alignment count of each segment bin to determine the first alignment count subjected to the alignment probability correction comprises:

generating a second relationship curve based on the normalized sliding window alignment count of each segment bin and the normalized first alignment count corresponding to each segment bin; and based on the second relationship curve, performing the alignment probability correction on the normalized first alignment count to obtain the first alignment count subjected to the alignment probability correction.

32. The method according to claim 31, wherein
before generating the second relationship curve based on the normalized sliding window alignment count of each segment bin and the normalized first alignment count corresponding to each segment bin, the method further comprises:

filtering the plurality of segment bins based on normalized sliding window alignment counts of the plurality of segment bins and obtaining segment bins whose normalized sliding window alignment counts are each not less than a first target threshold to enable the second relationship curve to be generated for the segment bins whose normalized sliding window alignment counts are each not less than the first target threshold;
or
based on the second relationship curve, performing the alignment probability correction on the normalized first alignment count to obtain the first alignment count subjected to the alignment probability correction comprises:

filtering the plurality of segment bins based on normalized sliding window alignment counts of the plurality of segment bins and retaining second relationship curve sections of the second relationship curve where normalized sliding window alignment counts are each not less than a first target threshold to obtain a second relationship curve after alignment probability filtering; and
based on the second relationship curve after alignment probability filtering, performing the alignment probability correction on the normalized sliding window alignment count to obtain the first alignment count subjected to the alignment probability correction.

33. The method according to any one of claims 22 to 32, wherein determining the first concentration of fetal DNA based on the first alignment counts of the plurality of segment bins comprises:

obtaining first training sample data, wherein each sample in the first training sample data is labeled with first feature values and a first target value, the first feature values are alignment counts, and the first target value is an actual concentration of fetal DNA;
based on a particular model structure, performing machine learning modeling on the first training sample data to obtain a first concentration quantitation model of fetal DNA; and
inputting the first alignment counts into the first concentration quantitation model of fetal DNA to obtain the first concentration of fetal DNA.

34. The method according to claim 33, wherein based on the particular model structure, performing the machine learning modeling on the first training sample data to obtain the first concentration quantitation model of fetal DNA comprises:

dividing the first training sample data into a training set and a test set;
performing the machine learning modeling based on the training set to obtain an initial model;

processing the test set based on the initial model to obtain a predicted concentration of fetal DNA in each test sample in the test set;

comparing the predicted concentration of fetal DNA with an actual concentration of fetal DNA in each test sample in the test set to obtain a comparison result; and

adjusting a model parameter of the initial model based on the comparison result to obtain the first concentration quantitation model of fetal DNA.

35. The method according to any one of claims 22 to 32, wherein determining the first concentration of fetal DNA based on the first alignment counts of the plurality of segment bins comprises:

inputting the first alignment counts into a first preset model to obtain an initial concentration of fetal DNA, wherein the first preset model is a linear relationship model built based on alignment results in second sample data and an initial concentration of fetal DNA corresponding to the alignment results, and the second sample data comprise the alignment results of alignment of sequencing reads of a cfDNA sample with the reference genome and the concentration of fetal DNA corresponding to the alignment results; and

correcting the initial concentration of fetal DNA based on a second preset model to obtain the first concentration of fetal DNA, wherein the second preset model is a model obtained after the first preset model is processed based on constants determined through linear fitting.

36. The method according to any one of claims 22 to 25, wherein based on the second alignment count of times each base site of the reference genome acts as the starting positions of alignment of the sequencing reads with the reference genome, determining the nucleosome center positions comprises:

determining the second alignment count of times each base site of the reference genome acts as the starting positions of alignment of the sequencing reads with the reference genome, and based on the second alignment count, calculating a nucleosome center score corresponding to each base site of the reference genome; and determining the nucleosome center positions based on nucleosome center scores corresponding to base sites of the reference genome and a center score screening threshold.

37. The method according to any one of claims 22 to 35, wherein determining the alignment results based on the first concentration of fetal DNA and the summed second alignment counts comprises:

performing dimensionality reduction on the summed second alignment counts corresponding to base sites within the nucleosome regions to obtain normalized second alignment counts subjected to the dimensionality reduction; and

determining the alignment results based on the first concentration of fetal DNA and the normalized second alignment counts subjected to the dimensionality reduction;

or

determining initial alignment results based on the first concentration of fetal DNA and the summed second alignment counts; and

performing dimensionality reduction on the initial alignment results, and determining initial alignment results subjected to the dimensionality reduction as the alignment results.

38. The method according to claim 36, wherein calculating the nucleosome center score corresponding to each base site of the reference genome comprises:

calculating a first count mean of second alignment counts of a first particular number of bases on the left and the first particular number of bases on the right of each base site of the reference genome;

calculating a second count mean of second alignment counts of a second particular number of bases on the left and the second particular number of bases on the right of each base site of the reference genome; and

determining the nucleosome center score corresponding to each base site according to the first count mean and the second count mean.

39. The method according to claim 38, wherein the nucleosome center score is calculated by the following formula:

the nucleosome center score =

$$\frac{count\ mean\ in\ abin[x-93,x-74+n]+count\ mean\ in\ a\ bin\ [x+93,x+74-n]}{count\ mean\ in\ a\ bin[x-73+n,x+73-n]},$$

wherein x represents the base site, [x-93, x-74+n] represents a bin from a site 93 nucleotides away from x on a side of x to a site (74-n) nucleotides away from x on the same side, [x+93, x+74-n] represents a bin from a site 93 nucleotides away from x on another side of x to a site (74-n) nucleotides away from x on the same side, [x-73+n, x+73-n] represents a bin from a site (73-n) nucleotides away from x on the side of x to a site (73-n) nucleotides away from x on the another side of x, and n represents a natural number less than or equal to 5.

40. The method according to any one of claims 36 to 39, wherein determining the nucleosome center scores based on the nucleosome center scores corresponding to the base sites of the reference genome and the center score screening threshold comprises:

   determining a maximum value of the nucleosome center scores and determining a first position corresponding to the maximum value in the reference genome;
   zeroing nucleosome center scores of bases of particular data on two sides of the first position, redetermining a maximum value from remaining nucleosome center scores after zeroing, and determining a position corresponding to the re-determined maximum value in the reference genome, until the remaining nucleosome center scores are each less than a second target threshold, and determining screened positions as candidate nucleosome center positions; and
   determining the nucleosome center positions based on the center score screening threshold and nucleosome center scores corresponding to the candidate nucleosome center positions.

41. The method according to any one of claims 22 to 39, wherein in a step of, based on the nucleosome center positions, performing the alignment and the summation on the second alignment counts at the corresponding positions within all the nucleosome regions, the corresponding positions within each nucleosome region comprise:
   a particular number of bases on the left of each of the nucleosome center positions and the particular number of bases on the right of each of the nucleosome center positions.

42. The method according to any one of claims 22 to 41, wherein determining the concentration of fetal DNA based on the alignment results comprises:

   acquiring second training sample data, wherein each sample in the second training sample data is labeled with feature values and a target value, the feature values are normalized second alignment counts subjected to the dimensionality reduction, and the target value is an actual concentration of fetal DNA;
   based on the second training sample data, building a second concentration quantitation model of fetal DNA; and
   inputting the alignment results into the second concentration quantitation model of fetal DNA to obtain the concentration of fetal DNA.

43. An apparatus for determining a concentration of fetal DNA, comprising:

   a first acquisition unit, which is configured to acquire a plurality of sequencing reads of a cell-free DNA (cfDNA) sample under test;
   a first alignment unit, which is configured to align the plurality of sequencing reads with a reference genome to obtain alignment results; and
   a first determination unit, which is configured to determine the concentration of fetal DNA based on the alignment results.

44. The apparatus according to claim 43, wherein the first alignment unit comprises:

   a first segmentation subunit, which is configured to segment the reference genome into a plurality of segment bins; and
   a first determination subunit, which is configured to determine, among the plurality of sequencing reads, a first alignment count of sequencing reads falling within each segment bin of the plurality of segment bins, and determine first alignment counts of the plurality of segment bins as the alignment results.

**45.** The apparatus according to claim 44, wherein the first segmentation subunit is configured to:

based on a preset segmentation length, segment the reference genome into a plurality of initial segment bins; and remove segment bins corresponding to a particular chromosome from the plurality of initial segment bins to obtain the plurality of segment bins;
or
remove a particular chromosome from the reference genome to obtain a removed reference genome; and based on a preset segmentation length, segment the removed reference genome into the plurality of segment bins.

**46.** The apparatus according to claim 44 or 45, further comprising:
a first correction unit, which is configured to correct the first alignment count to obtain a corrected first alignment count, wherein corrected first alignment counts of the plurality of segment bins are determined as the alignment results.

**47.** The apparatus according to claim 46, wherein the first correction unit comprises:

a first processing subunit, which is configured to, based on the first alignment count of each segment bin and a mean of the first alignment counts of the plurality of segment bins, normalize the first alignment count of each segment bin to obtain a normalized first alignment count of each segment bin; and
a guanine-cytosine (GC) correction subunit, which is configured to perform GC correction on the normalized first alignment count of each segment bin to obtain a GC-corrected first alignment count.

**48.** The apparatus according to claim 47, wherein the GC correction subunit is configured to:

generate a first relationship curve based on a GC content and the normalized first alignment count of each segment bin; and
based on the first relationship curve, perform the GC correction on the normalized first alignment count to obtain the GC-corrected first alignment count.

**49.** The apparatus according to claim 47, wherein the first correction unit further comprises:

a cutting subunit, which is configured to cut the reference genome based on a particular sliding window length to obtain sequence cuts, align the sequence cuts with the reference genome, count a first alignment count of sequence cuts falling within each segment bin, and determine the first alignment count as a sliding window alignment count of each segment bin;
a second determination subunit, which is configured to, based on the sliding window alignment count, determine a normalized sliding window alignment count of each segment bin; and
a third determination subunit, which is configured to perform alignment probability correction based on the normalized sliding window alignment count and the normalized first alignment count of each segment bin to determine a first alignment count subjected to the alignment probability correction.

**50.** The apparatus according to claim 43, wherein the first determination unit comprises:

a first sample acquisition subunit, which is configured to obtain first training sample data, wherein each sample in the first training sample data is labeled with first feature values and a first target value, the first feature values are alignment counts, and the first target value is an actual concentration of fetal DNA;
a first modeling subunit, which is configured to, based on a particular model structure, perform machine learning modeling on the first training sample data to obtain a first concentration quantitation model of fetal DNA; and
a model processing subunit, which is configured to input the alignment results into the first concentration quantitation model of fetal DNA to obtain a first concentration of fetal DNA and determine the first concentration of fetal DNA as the concentration of fetal DNA.

**51.** The apparatus according to any one of claims 43 to 49, wherein the first determination unit is further configured to:

input the alignment results into a first preset model to obtain an initial concentration of fetal DNA, wherein the first preset model is a linear relationship model built based on alignment results in second sample data and an initial concentration of fetal DNA corresponding to the alignment results, and the second sample data comprise the alignment results of alignment of sequencing reads of a cfDNA sample with the reference genome and the concentration of fetal DNA corresponding to the alignment results; and

correct the initial concentration of fetal DNA based on a second preset model to obtain the concentration of fetal DNA, wherein the second preset model is a model obtained after the first preset model is processed based on constants determined through linear fitting.

52. The apparatus according to claim 43, wherein the first alignment unit is configured to:

determine a second alignment count of times each base site of the reference genome acts as starting positions of alignment of sequencing reads with the reference genome, and based on the second alignment count, calculate a nucleosome center score corresponding to each base site of the reference genome;

determine nucleosome center positions based on nucleosome center scores corresponding to base sites of the reference genome and a center score screening threshold;

based on the nucleosome center positions, perform alignment and summation on second alignment counts at corresponding positions within all nucleosome regions to obtain summed second alignment counts; and

perform dimensionality reduction on the summed second alignment counts to obtain normalized second alignment counts subjected to the dimensionality reduction, and determine the normalized second alignment counts subjected to the dimensionality reduction as the alignment results;

wherein calculating the nucleosome center score corresponding to each base site of the reference genome comprises:

calculating a first count mean of second alignment counts of a first particular number of bases on the left and the first particular number of bases on the right of each base site of the reference genome;

calculating a second count mean of second alignment counts of a second particular number of bases on the left and the second particular number of bases on the right of each base site of the reference genome; and

determining the nucleosome center score corresponding to each base site according to the first count mean and the second count mean.

53. The apparatus according to claim 52, wherein the first determination unit is further configured to:

acquire second training sample data, wherein each sample in the second training sample data is labeled with feature values and a target value, the feature values are normalized second alignment counts subjected to the dimensionality reduction, and the target value is an actual concentration of fetal DNA;

based on the second training sample data, build a second concentration quantitation model of fetal DNA; and

input the alignment results into the second concentration quantitation model of fetal DNA to obtain the concentration of fetal DNA.

54. An apparatus for determining a concentration of fetal DNA, comprising:

a second acquisition unit, which is configured to acquire a plurality of sequencing reads of a cell-free DNA (cfDNA) sample under test;

a second determination unit, which is configured to segment a reference genome into a plurality of segment bins and determine, among the plurality of sequencing reads, a first alignment count of sequencing reads falling within each segment bin of the plurality of segment bins;

a third determination unit, which is configured to determine a first concentration of fetal DNA based on first alignment counts of the plurality of segment bins;

a fourth determination unit, which is configured to, based on a second alignment count of times each base site of the reference genome acts as starting positions of alignment of sequencing reads with the reference genome, determine nucleosome center positions;

a processing unit, which is configured to, based on the nucleosome center positions, perform alignment and summation on second alignment counts at corresponding positions within all nucleosome regions to obtain summed second alignment counts;

a fifth determination unit, which is configured to determine alignment results based on the first concentration of fetal DNA and the summed second alignment counts; and

a sixth determination unit, which is configured to determine the concentration of fetal DNA based on the alignment results.

55. The apparatus according to claim 54, wherein the second determination unit comprises:
a second segmentation subunit, which is configured to:

based on a preset segmentation length, segment the reference genome into a plurality of initial segment bins; and remove segment bins corresponding to a particular chromosome from the plurality of initial segment bins to obtain the plurality of segment bins;

or

remove a particular chromosome from the reference genome to obtain a removed reference genome; and based on a preset segmentation length, segment the removed reference genome into the plurality of segment bins.

56. The apparatus according to claim 54 or 55, further comprising:

a second correction unit, which is configured to correct the first alignment count to obtain a corrected first alignment count, wherein the first concentration of fetal DNA is determined based on corrected first alignment counts of the plurality of segment bins.

57. The apparatus according to claim 56, wherein the second correction unit comprises:

a second processing subunit, which is configured to, based on the first alignment count of each segment bin and a mean of the first alignment counts of the plurality of segment bins, normalize the first alignment count of each segment bin to obtain a normalized first alignment count of each segment bin; and

a guanine-cytosine (GC) correction subunit, which is configured to perform GC correction on the normalized first alignment count of each segment bin to obtain a GC-corrected first alignment count.

58. The apparatus according to claim 57, wherein the GC correction subunit is configured to:

based on a GC content and the normalized first alignment count of each segment bin, generate a first relationship curve matching a particular coordinate system; and

based on the first relationship curve, perform the GC correction on the normalized first alignment count to obtain the GC-corrected first alignment count.

59. The apparatus according to any one of claims 56 to 58, wherein the second correction subunit is further configured to:

cut the reference genome based on a particular sliding window length to obtain sequence cuts, align the sequence cuts with the reference genome, count a first alignment count of sequence cuts falling within each segment bin, and determine the first alignment count as a sliding window alignment count of each segment bin;

based on the sliding window alignment count, determine a normalized sliding window alignment count of each segment bin; and

perform alignment probability correction based on the normalized sliding window alignment count and the normalized first alignment count of each segment bin to determine a first alignment count subjected to the alignment probability correction.

60. The apparatus according to any one of claims 54 to 59, wherein the third determination unit is configured to:

obtain first training sample data, wherein each sample in the first training sample data is labeled with first feature values and a first target value, the first feature values are alignment counts, and the first target value is an actual concentration of fetal DNA;

based on a particular model structure, perform machine learning modeling on the first training sample data to obtain a first concentration quantitation model of fetal DNA; and

input the first alignment counts into the first concentration quantitation model of fetal DNA to obtain the first concentration of fetal DNA.

61. The apparatus according to any one of claims 54 to 59, wherein the third determination unit is further configured to:

input the first alignment counts into a first preset model to obtain an initial concentration of fetal DNA, wherein the first preset model is a linear relationship model built based on alignment results in second sample data and an initial concentration of fetal DNA corresponding to the alignment results, and the second sample data comprise the alignment results of alignment of sequencing reads of a cfDNA sample with the reference genome and the concentration of fetal DNA corresponding to the alignment results; and

correct the initial concentration of fetal DNA based on a second preset model to obtain the first concentration of fetal DNA, wherein the second preset model is a model obtained after the first preset model is processed based on constants determined through linear fitting.

62. The apparatus according to claim 54, wherein the fourth determination unit comprises:

a score calculation subunit, which is configured to determine the second alignment count of times each base site of the reference genome acts as the starting positions of alignment of the sequencing reads with the reference genome, and based on the second alignment count, calculate a nucleosome center score corresponding to each base site of the reference genome; and
a center position determination subunit, which is configured to determine the nucleosome center positions based on nucleosome center scores corresponding to base sites of the reference genome and a center score screening threshold.

63. The apparatus according to claim 62, wherein the fifth determination unit is configured to:

perform dimensionality reduction on the summed second alignment counts corresponding to base sites within the nucleosome regions to obtain normalized second alignment counts subjected to the dimensionality reduction; and
determine the alignment results based on the first concentration of fetal DNA and the normalized second alignment counts subjected to the dimensionality reduction;
or
determine initial alignment results based on the first concentration of fetal DNA and the summed second alignment counts; and
perform dimensionality reduction on the initial alignment results, and determine initial alignment results subjected to the dimensionality reduction as the alignment results.

Acquire sequencing reads of a cfDNA sample under test

S101

Align the sequencing reads with a reference genome to obtain alignment results

S102

Determine a fetal concentration based on the alignment results

S103

**FIG. 1**

**FIG. 2**

FIG. 3

FIG. 4

FIG. 5

FIG. 6

FIG. 7

Acquire sequencing reads of a cfDNA sample under test — S201

Segment a reference genome into multiple segment bins, and determine a first alignment count of sequencing reads falling within each segment bin — S202

Determine a first fetal concentration based on first alignment counts of the multiple segment bins — S203

Based on a second alignment count of sequencing reads starting to align with the reference genome from each base site of the reference genome, determine nucleosome center positions — S204

Based on the nucleosome center positions, perform alignment and summation on second alignment counts at corresponding positions within all nucleosome regions to obtain summed second alignment counts — S205

Determine alignment results based on the first fetal concentration and the summed second alignment counts — S206

Determine a fetal concentration based on the alignment results — S207

FIG. 8

| First acquisition unit ⟋10 | First alignment unit ⟋11 | First determination unit ⟋12 |

**FIG. 9**

Second acquisition unit ⟋20

Second determination unit ⟋21

Third determination unit ⟋22

Fourth determination unit ⟋23

Processing unit ⟋24

Fifth determination unit ⟋25

Sixth determination unit ⟋26

**FIG. 10**

## INTERNATIONAL SEARCH REPORT

| International application No. |
|---|
| **PCT/CN2023/142606** |

**A. CLASSIFICATION OF SUBJECT MATTER**

C12Q1/6869(2018.01)i; C12Q1/6888(2018.01)i; G16B30/10(2019.01)i

According to International Patent Classification (IPC) or to both national classification and IPC

**B. FIELDS SEARCHED**

Minimum documentation searched (classification system followed by classification symbols)

IPC: C12Q; G16B

Documentation searched other than minimum documentation to the extent that such documents are included in the fields searched

Electronic data base consulted during the international search (name of data base and, where practicable, search terms used)

CNTXT, WPABSC, ENTXTC, ENTXT, USTXT, WOTXT, CJFD, VEN, CNKI, DWPI, 万方, WANFANG, 百度, BAIDU, bing, PUBMED, ISI Web of Knowledge, 读秀, DUXIU: 胎儿, DNA, 浓度, cfDNA, 测序, 比对, 校正, GC校正, 归一化, 基因组, 分段, 分窗, 滑窗, 对齐, 加和, 核小体, 排除, 剔除, 去除, X染色体, Y染色体, 线粒体染色体, 13号染色体, 18号染色体, 21号染色体, 无创产前基因检测, Non-Invasive Prenatal Test, NIPT, 机器学习, 模型, 测试, 样本, fetus, concentration, sequenc +, alignment, correction, normalization, genome, segment, windowing, sliding window, addition, nucleosome, exclusion, culling, removal, X chromosome, Y chromosome, mitochondrial chromosome, chromosome 13, chromosome 18, chromosome 21, non-invasive prenatal genetic test, Non-Invasive Prenatal Test, NIPT, machine learning, model, test, sample.

**C. DOCUMENTS CONSIDERED TO BE RELEVANT**

| Category* | Citation of document, with indication, where appropriate, of the relevant passages | Relevant to claim No. |
|---|---|---|
| PX | CN 117106870 A (GENEMIND BIOSCIENCES CO., LTD.) 24 November 2023 (2023-11-24) claims 1-10, and description, paragraphs 4-452 | 1-63 |
| X | WO 2021026828 A1 (SHENZHEN BGI GENOMICS CO., LTD.) 18 February 2021 (2021-02-18) claims 1-20, and description, pages 1-2 and 5-9 | 1-2, 22, 43, 54, 62-63 |
| Y | WO 2021026828 A1 (SHENZHEN BGI GENOMICS CO., LTD.) 18 February 2021 (2021-02-18) claims 1-20, and description, pages 1-2 and 5-9 | 3-17, 19-21, 23-38, 40-42, 44-61 |
| Y | CN 110770341 A (QUEST DIAGNOSTICS INVESTMENTS LLC) 07 February 2020 (2020-02-07) description, paragraphs 24, 44 and 116-144 | 3-17, 19-21, 23-38, 40-42, 44-61 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **21 March 2024** | **01 April 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2023/142606** |

### C. DOCUMENTS CONSIDERED TO BE RELEVANT

| Category* | Citation of document, with indication, where appropriate, of the relevant passages | Relevant to claim No. |
|---|---|---|
| Y | CN 110970089 A (USCI MEDICAL DEVICES CO., LTD.) 07 April 2020 (2020-04-07) claims 1-17, and description, paragraph 68 | 44-53 |
| A | CN 114945685 A (SHENZHEN BGI LIFE SCIENCES RESEARCH INSTITUTE) 26 August 2022 (2022-08-26) entire document | 1-63 |
| A | US 2021020314 A1 (JUNO DIAGNOSTICS, INC.) 21 January 2021 (2021-01-21) entire document | 1-63 |
| A | WO 2022140579 A1 (PROGENITY, INC.) 30 June 2022 (2022-06-30) entire document | 1-63 |
| A | WONG, F. C. K. et al. "Cell-free DNA in maternal plasma and serum: A comparison of quantity, quality and tissue origin using genomic and epigenomic approaches" *Clinical Biochemistry*, 09 September 2016 (2016-09-09), pages 1379-1386 | 1-63 |
| A | 刘弘泰等 (LIU, Hongtai et al.). "孕妇血浆中胎儿游离DNA浓度检测新方法 (New Detection Method of Cell-Free Fetal DNA Fraction in Maternal Plasma)" *武汉大学学报(医学版) (Medical Journal of Wuhan University)*, Vol. 38, No. 3, 31 May 2017 (2017-05-31), pages 435-438 | 1-63 |

Form PCT/ISA/210 (second sheet) (July 2022)

## INTERNATIONAL SEARCH REPORT
### Information on patent family members

International application No.

**PCT/CN2023/142606**

| Patent document cited in search report | | | Publication date (day/month/year) | Patent family member(s) | | | Publication date (day/month/year) |
|---|---|---|---|---|---|---|---|
| CN | 117106870 | A | 24 November 2023 | None | | | |
| WO | 2021026828 | A1 | 18 February 2021 | ES | 2942363 | T3 | 31 May 2023 |
| | | | | DK | 3916105 | T3 | 17 April 2023 |
| | | | | PL | 3916105 | T3 | 26 June 2023 |
| | | | | IL | 289007 | A | 01 February 2022 |
| | | | | EP | 3916105 | A1 | 01 December 2021 |
| | | | | EP | 3916105 | A4 | 06 April 2022 |
| | | | | EP | 3916105 | B1 | 25 January 2023 |
| | | | | HUE | 061561 | T2 | 28 July 2023 |
| CN | 110770341 | A | 07 February 2020 | MX | 2019008320 | A | 09 September 2019 |
| | | | | WO | 2018132400 | A1 | 19 July 2018 |
| | | | | US | 2020255896 | A1 | 13 August 2020 |
| | | | | CA | 3049442 | A1 | 19 July 2018 |
| | | | | BR | 112019014208 | A2 | 17 March 2020 |
| | | | | EP | 3568472 | A1 | 20 November 2019 |
| | | | | EP | 3568472 | A4 | 12 August 2020 |
| CN | 110970089 | A | 07 April 2020 | None | | | |
| CN | 114945685 | A | 26 August 2022 | None | | | |
| US | 2021020314 | A1 | 21 January 2021 | CA | 3095030 | A1 | 03 October 2019 |
| | | | | AU | 2019244115 | A1 | 19 November 2020 |
| | | | | WO | 2019191319 | A1 | 03 October 2019 |
| WO | 2022140579 | A1 | 30 June 2022 | None | | | |

Form PCT/ISA/210 (patent family annex) (July 2022)

**REFERENCES CITED IN THE DESCRIPTION**

**Patent documents cited in the description**

- CN 202211727280